# EUROPEAN PATENT APPLICATION

(11) **EP 3 715 360 A1**
(43) Date of publication of application: **30.09.2020**
(21) Application number: 19166308.7
(22) Date of filing: 29.03.2019
(51) Int. Cl.: C07K 14/195

(54) **OPTOGENETIC SWITCHES IN BACTERIA**

(71) Applicant: Max-Planck-Gesellschaft zur Förderung der Wissenschaften e.V., 80539 München (DE)
(72) Inventor: The designation of the inventor has not yet been filed
(74) Representative: Virnekäs, Bernhard

(57) **Abstract**

The present invention relates to a recombinant bacterium wherein said bacterium comprises an optogenetic interaction switch to control cellular functions, in particular wherein said bacterium is a recombinant gram-negative bacterium comprising a type III secretion system, wherein the activity of said type III secretion system is light-dependent, and to methods for controlling cellular functions in a bacterium using such an optogenetic interaction switch.

## Description

The present invention relates to a recombinant bacterium wherein said bacterium comprises an optogenetic interaction switch to control cellular functions, in particular wherein said bacterium is a recombinant gram-negative bacterium comprising a type III secretion system, wherein said type III secretion system is light-dependent, and to methods for controlling cellular functions in a bacterium using such an optogenetic interaction switch.

### BACKGROUND

Optogenetics provides a toolbox for combining optical and genetic methods to achieve precisely controllable reversible gain or loss of protein function in living cells or tissues. It allows fast (within milliseconds) and specific (to single proteins) control of defined events in biological systems without any major perturbation of the biological target system (Deisseroth, 2011). These abilities can give optogenetic approaches an advantage over knockdown, overexpression, or mutant strain analysis, which often display slower activation and a broader effect (Toettcher et al, 2011a). Most optogenetic tools are based on modified opsins, rhodopsins or phototropins, which are light-inducible proteins that undergo a conformational change upon irradiation (Deisseroth, 2011; Wang et al, 2016).

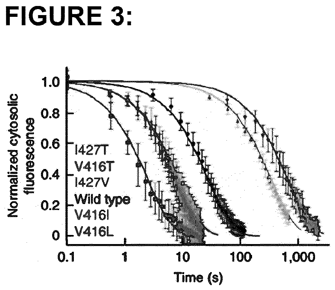

Optogenetic protein interaction switches use light-induced conformational changes of specific proteins, often light-oxygen-voltage (LOV) domain proteins, to control protein interactions by light ((Kawano et al, 2015; Guntas et al, 2015; Wang et al, 2016)). They usually consist of two identical or different proteins whose affinity is strongly altered upon irradiation by light of a certain wavelength. Mutations of specific amino acids in the optogenetic proteins can modulate the binding affinity and corresponding dissociation or return rates from a few seconds to several minutes (Kawano et al, 2015; Zimmerman et al, 2016; Wang et al, 2016) (Fig. 3).

Currently, optogenetic systems are mainly studied in mammalian cells (mostly in neuroscience) (Mukherjee et al, 2017), and while there is an increasing interest in establishing these systems also in bacteria, for example as an optogenetic promoter system to regulate gene expression in *E. coli* (Jayaraman et al, 2016), no optogenetic system has been developed so far to regulate bacterial cell functions by light-dependent control over protein-protein interactions.

Thus, there was an unmet need to establish system that could permit the light-based modulation of bacterial cellular functions.

### SUMMARY OF THE INVENTION

The present invention is based on the surprising observation, that by anchoring a member of a light-dependent protein binding pair, for example to the cytoplasmic membrane, the activity of a protein of interest, which causes or modulates a cellular function of the bacterial host cell, and which is coupled to the other member of the light-regulated protein binding pair, can be switched on and off by light-induced cleavage and reformation of the light-dependent protein binding pair.

In a first aspect, the present invention relates to recombinant gram-negative bacterium comprising a type III secretion system, wherein said type III secretion system is light-dependent.

In a second aspect, the present invention relates to a method for modifying the translocation of one or more cargo proteins from a recombinant gram-negative bacterium, comprising the steps of (i) culturing a recombinant gram-negative bacterium comprising a light-dependent type III secretion system of the present invention under a first light condition, and (ii) culturing said recombinant gram-negative bacterium under a second light condition, wherein the change from said first light condition to said second light condition modifies the translocation activity of said light-dependent type III secretion system.

In a third aspect, the present invention relates to a recombinant bacterium wherein said bacterium comprises an optogenetic interaction switch to control one or more cellular functions.

In a fourth aspect, the present invention relates to method for modifying at least one cellular function of a recombinant bacterium, comprising the steps of (i) culturing a recombinant bacterium comprising an optogenetic interaction switch of the third aspect of the present invention under a first light condition, and (ii) culturing said recombinant bacterium under a second light condition, wherein the change from said first light condition to said second light condition modifies said at least one cellular function.

### FIGURES

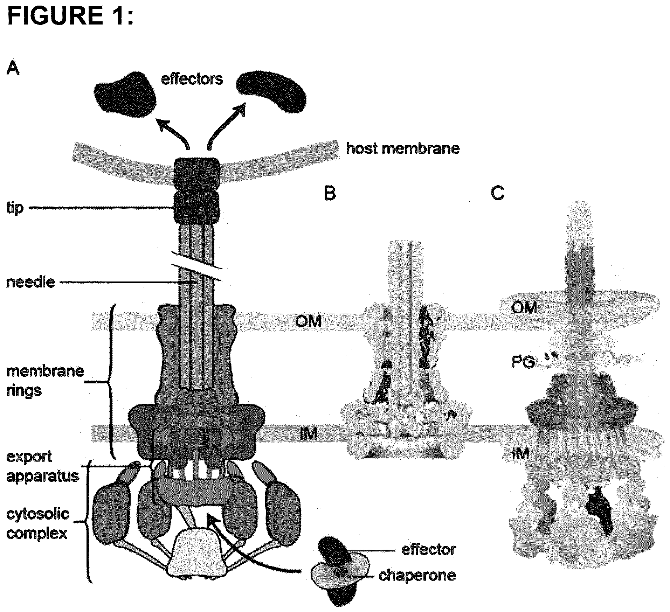

**Figure 1** shows the structure and composition of the type III secretion system. (A) Schematic representation of the T3SS injectisome (modified from (Diepold & Wagner, 2014)). The main substructures are indicated on the left, see main text for details. (B) Cut-through surface representation of a 3D reconstruction of parts of the Salmonella SPI-1 injectisome based on cryo-electron microscopy data (Schraidt & Marlovits, 2011). (C) 3D reconstruction of the Salmonella SPI-1 injectisome based on cryo-electron tomography data (Hu et al, 2017). OM, outer membrane; IM, inner membrane; PG, peptidoglycan layer.

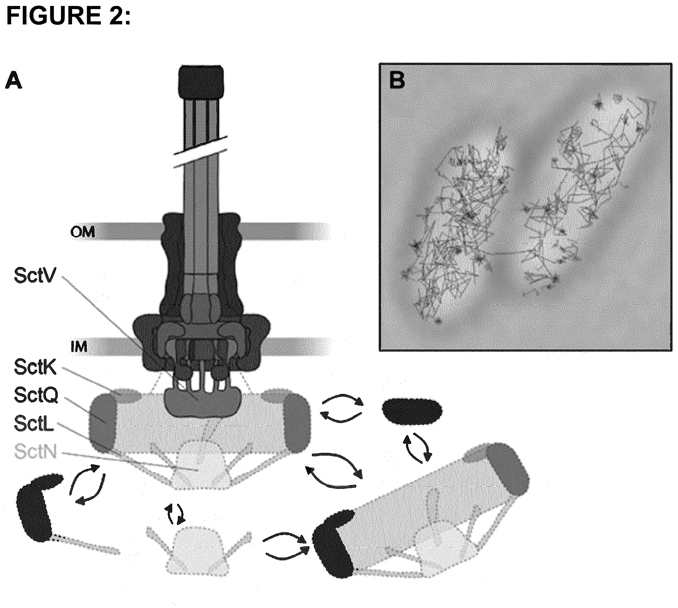

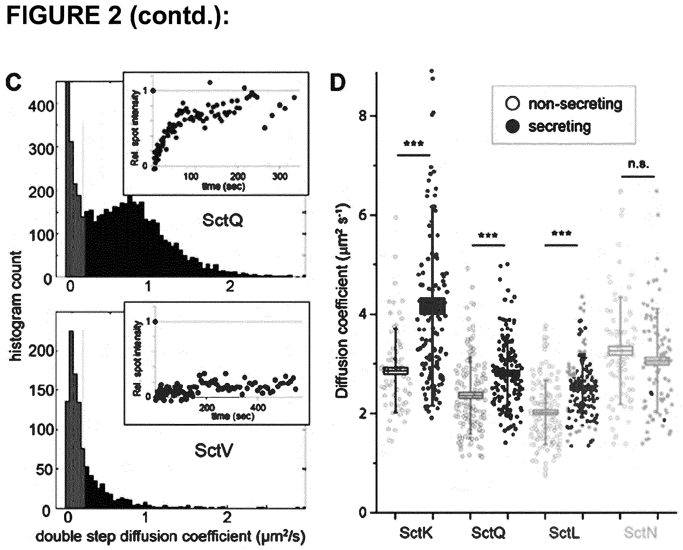

**Figure 2** shows that the cytosolic components of the T3SS are in constant exchange between the injectisome and a cytosolic pool. (A) Representation of the bound and possible unbound states of the cytosolic T3SS components. Protein names of the cytosolic components as well as the the major export apparatus protein SctV, used as a control, are displayed on the left. (B) Single-molecule tracks of PAmCherry-SctQ in live secreting Y. *enterocolitica.* Red, static (bound) proteins; blue, diffusing (unbound) proteins. (C) Distribution of diffusion coefficients for PAmCherry-labelled SctQ (top) and SctV (bottom). Insets: Fluorescence recovery after photobleaching (representative curves) showing the exchange of SctQ, but not SctV at the injectisome. (D). Upon induction of secretion (filled bars and circles), the cytosolic components SctK, SctQ, and SctL - but not the ATPase SctN - diffuse significantly faster within live bacteria (boxes, standard error of the mean; whiskers, standard deviation; ***, p<1 E-6; n.s., no statistically significant difference). B-C modified from (Diepold et al, 2015); D modified from (Diepold et al, 2017).
**Figure 3** shows an example for the modulation of dissociation rates caused by mutations in the LOV system. The binding affinities and return rates of the optogenetic systems, here as an example for the LOV system, can be modulated from few seconds to many minutes by several mutations (Wang et al, 2016).

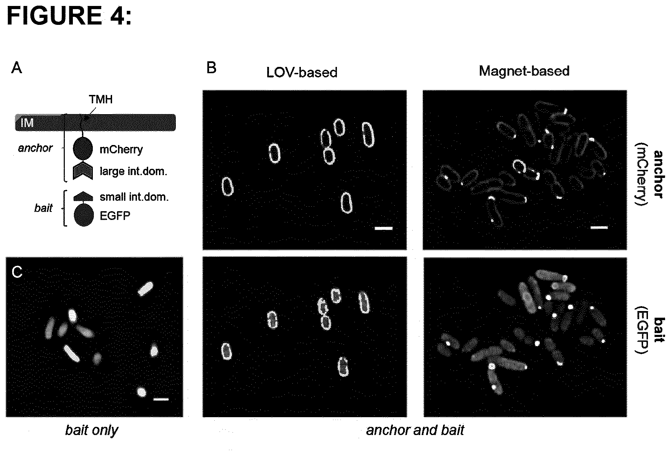

**Figure 4** shows the localization of fluorescently labeled optogenetic interaction domains in Y. *enterocolitica.* (A) Scheme of optogenetic membrane sequestration of bait proteins. (B) Localization of the mCherry-labeled anchor proteins (top) and the EGFP-labeled bait proteins (bottom) for the LOV (left) and Magnet-based system (right). (C) Cytosolic localization of the LOV bait protein Zdk1-EGFP in absence of the membrane anchor. Bacteria were incubated at ambient light, and imaged with an inverted fluorescence microscope. TMH, extended transmembrane helix; int.dom., interaction domain. Scale bars, 2 µm.

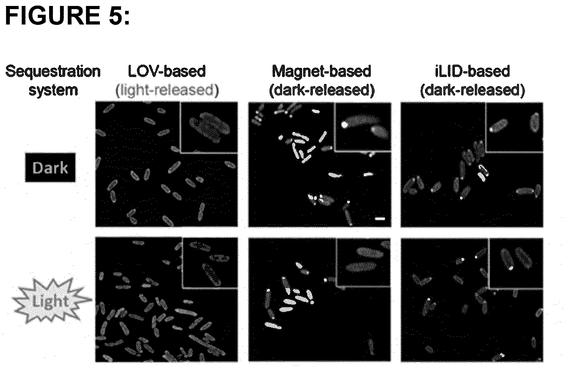

**Figure 5** shows the Influence of blue light on the localization of bait proteins in Y. *enterocolitica.* Visualization of the localization of mCherry-labeled bait proteins in fixed *Y. enterocolitica* samples expressing both interaction partners of the indicated systems. Samples were handled as described in the main text, and fixed as described in material and methods. Scale bar, 2 µm.

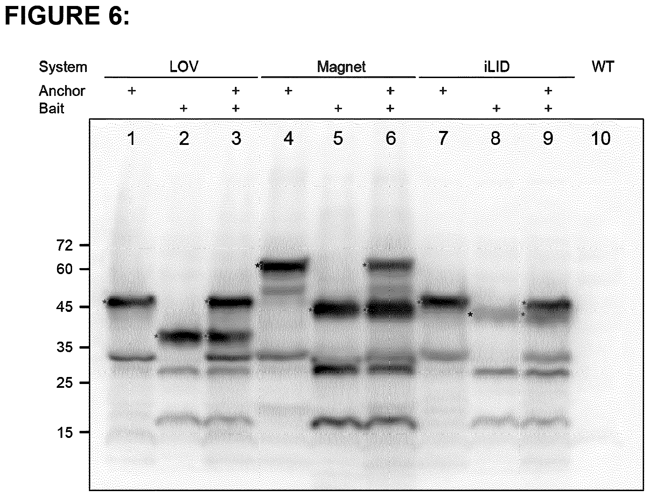

**Figure 6** shows a Western blot (anti-mCherry) with optogenetic fusion proteins to test expression level in Y. *enterocolitica.* Total cellular proteins from 1.5^{∗}10⁸ *Y. enterocolitica* expressing either the membrane anchors in fusion with mCherry (expected size indicated by upper stars), the bait tagged with mCherry (expected size indicated by lower stars), or both proteins for each indicated system, as indicated. As a control, an untagged Y. *enterocolitica* strain, dHOPEMTasd (WT), was used. Protein size in kDa indicated on left side.

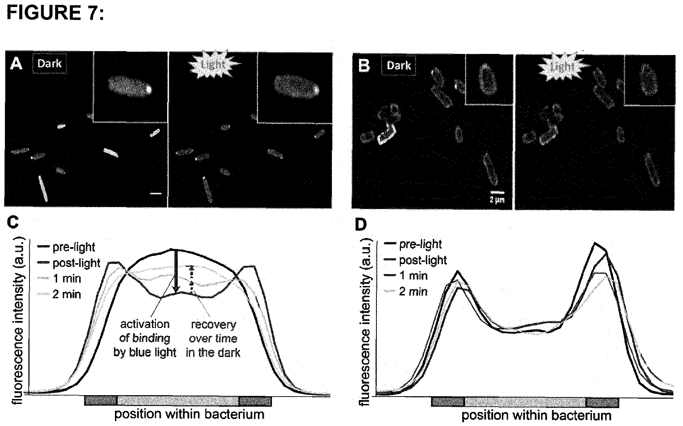

**Figure 7** shows the activation and recovery kinetics of optogenetic sequestration systems. (A/B) Fluorescence micrographs of mCherry-labeled bait proteins in the iLID-based (A) and LOV-based (B) sequestration system, before (left) and directly after (right) illumination with blue light. (C/D) Fluorescence quantification across bacteria over time (n=25) in the iLID-based (C) and LOV-based (D) sequestration system; dark grey: membrane, light grey: cytosol.

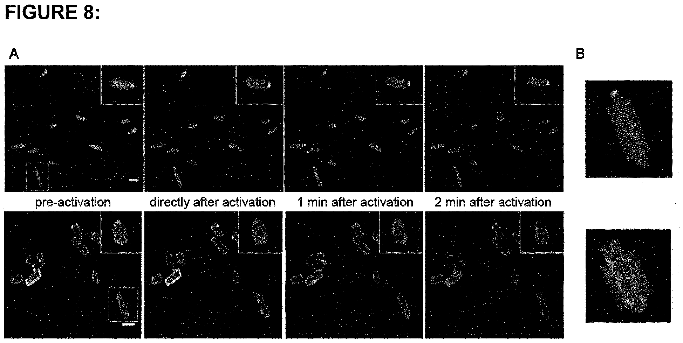

**Figure 8** shows the effect of illumination and recovery kinetics in the light-induced sequestration systems. Localization of mCherry-labelled bait proteins in live Y. *enterocolitica* was imaged at the indicated timepoints for the iLID-based sequestration system (A), and the LOV-based sequestration system. For activation with blue light, 0.1 sec illumination at a wavelength of around 480 nm was used. Pictures were taken every minute after activation. Yellow boxes highlight the bacteria analyzed in Fig. 7. (B) Schematic representation of the line scans used for the fluorescence intensity profiles in Fig. 7. Scale bars, 2 µm.

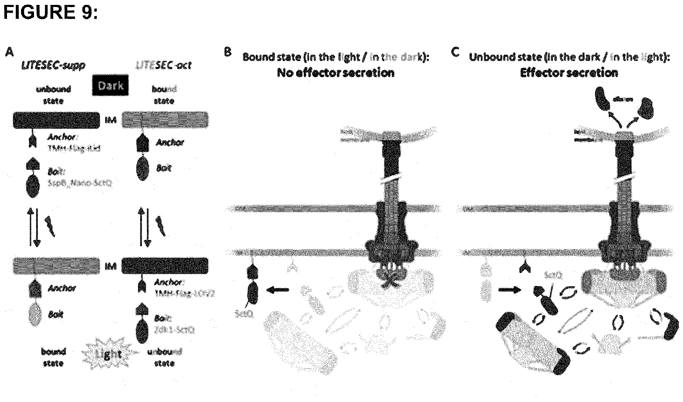

**Figure 9** shows the working principle of the LITESEC systems - light-controlled activation and deactivation of protein translocation by the type III secretion system. (A) Different states of the bait and anchor proteins in dark and light conditions. In the LITESEC-supp system (left side, red text and symbols), the bait protein, a fusion of the interacting domain SspB_Nano and the essential T3SS component SctQ, is tethered to the inner membrane (IM) by a membrane anchor, of fusion of a transmembrane helix (TMH) and the other interacting domain, iLID, in the light, and gets released in the dark. Conversely, in the LITESEC-act system (right side, green text and symbols), the bait protein, a fusion of the interacting domain Zdk1 and the essential T3SS component SctQ, is tethered to the membrane anchor, a TMH fusion of the interaction partner, LOV2, in the dark, and gets released by illumination. (B) In the bound state, the bait-SctQ fusion protein is tethered to the membrane anchor. Its subsequent absence in the cytosol prevents effector secretion. (C) In the unbound state, effector translocation by the T3SS can occur by the functional interaction of the unbound bait-SctQ fusion with the T3SS.

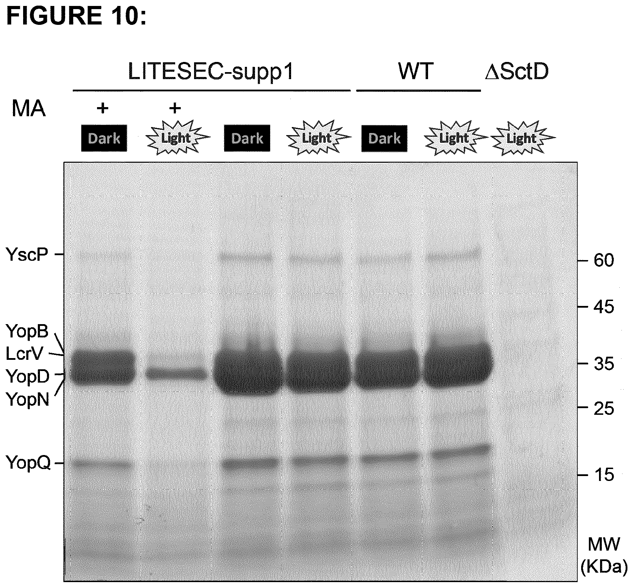

**Figure 10** shows the secretion of effector proteins by the type III secretion system can be controlled by light. In vitro secretion assay showing light-dependent export of T3SS substrates (indicated on the left) in the LITESEC-supp1 strain. Proteins secreted by 3^{∗}10⁹ bacteria during a 180 min incubation period were precipitated and analyzed by SDS-PAGE. The strain lacking a membrane anchor (MA), the wild-type strain ΔHOPEMTasd and the T3SS-negative strain ΔSctD were used as controls. This experiment was repeated at least 3 times with similar results. MW, molecular weight in kDa (right side).

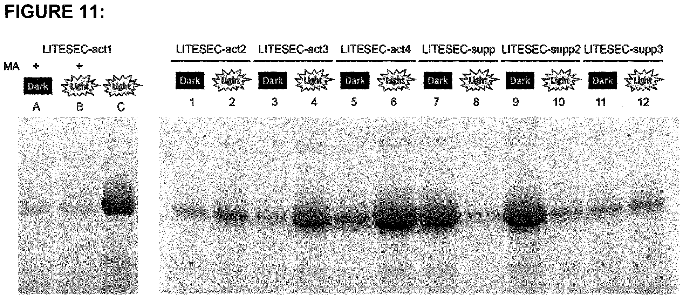

**Figure 11** shows the improved secretion efficiency and light responsiveness in evolved versions of the LITESEC strains. In vitro secretion assay showing light-dependent export of translocator proteins (LcrV (SctA), YopB (SctE), YopD (SctB), size of ∼ 35 kDa) (Diepold et al, 2011) in the LITESEC-act1 strain (left panel), and in various improved versions of the LITESEC strains (right panel). Proteins secreted by 3^{∗}10⁹ bacteria during a 180 min incubation period were precipitated and analyzed by SDS-PAGE. MA, presence of membrane anchor (membrane anchors are present in all lanes on the right panel).

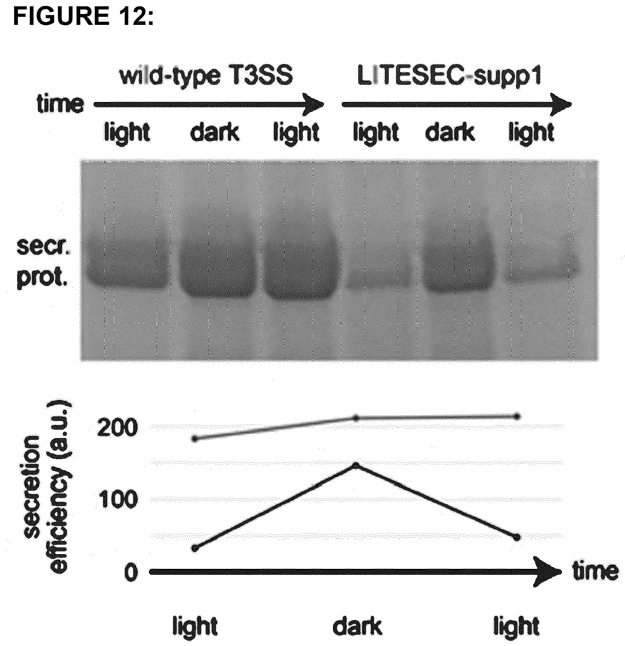

**Figure 12** shows that secretion of effector proteins can be controlled by light over time. Time course showing constant secretion of effector proteins (secr. prot.) in a wild-type strain (left, grey) and light-induced increase and decrease of secretion in the LITESEC-supp1 strain (right, red). After induction of the T3SS by temperature shift to 37°C, samples were incubated subsequently under light, dark, and light conditions for 60 min each. At the end of each 60 min interval, samples were taken, and the bacteria were washed and resuspended in fresh pre-warmed media. Proteins secreted by 3^{∗}10⁹ bacteria were precipitated and analyzed by SDS-PAGE. Top, visualization of secreted proteins, bottom: quantification of secretion.

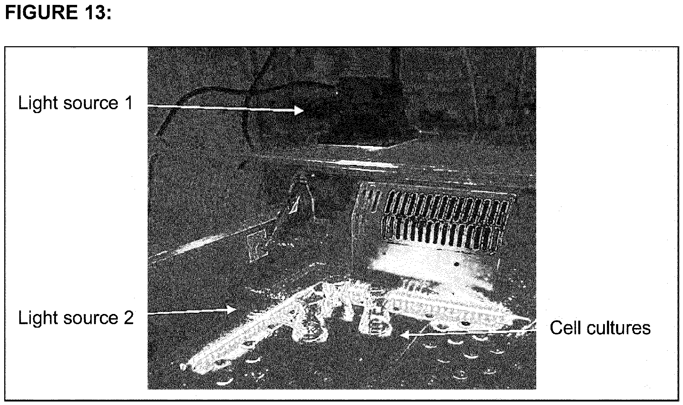

**Figure 13** shows the optogenetic experimental setup. The optogenetic experimental setup consists of two blue light sources that were placed around the cell cultures. Light source 1 was a "globo lighting 10 W LED 9 V 34118S" - (Globo Lighting GmbH (St. Peter, A)), Light source 2 was a "Rolux LED-Leiste DF-7024-12 V 1.5 W" - (Rolux Leuchten GmbH (Weyhe, Germany)). Cell cultures were cultivated at 37° C.

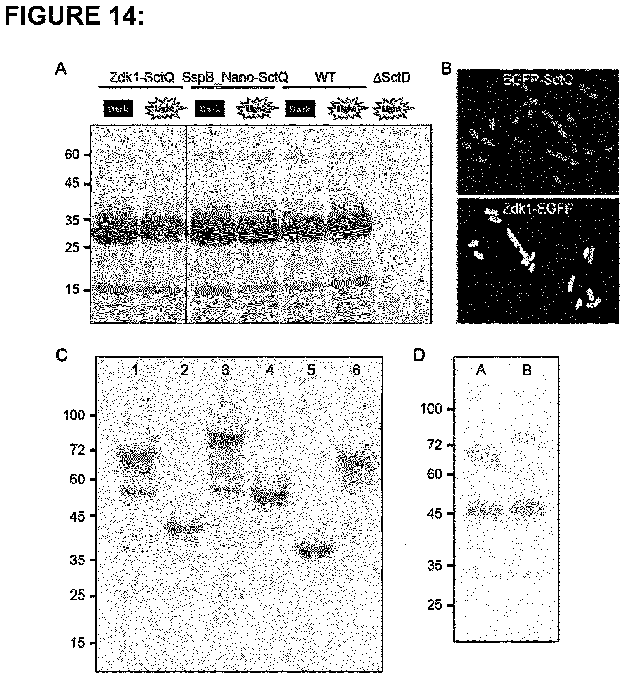

**Figure 14** shows that the fusion proteins that were used are stable, functional, and expressed at levels that are suitable for the optogenetic sequestration. (A) In vitro secretion assay showing export of T3SS substrates in the strains expressing the indicated SctQ fusions instead of wild-type SctQ under light and dark condition. Proteins secreted by 3^{∗}10⁹ bacteria during a 180 min incubation period were precipitated and analyzed by SDS-PAGE. The wild-type strain ΔHOPEMTasd and the T3SS-negative strain ΔSctD were used as controls. Horizontal line indicates the omission of intermediate lanes. Note that the displayed gel partially overlaps with the gel shown in Fig. 10. (B) Fluorescence intensity of the bait proteins EGFP-SctQ (left, expressed from its native locus on the virulence plasmid) and Zdk1-EGFP (left, expressed from a pACYC184-based plasmid), imaged and processed identically to allow comparison. The EGFP-SctQ strain has an additional deletion in SctL to prevent the binding of SctQ to injectisomes, for better comparability. (C) Western blot anti-SctQ of the used bait-SctQ and bait-mCherry-SctQ fusion proteins, expressed from the native genetic locus on the virulence plasmid. Control strains, dHOPEMTasd (wild-type SctQ) and AD4324 (mCherry-SctQ). Bait-SctQ fusions without mCherry (lanes 2, 4) showed no cleavage. Bait-mCherry-SctQ fusion proteins (lanes 1, 3) showed a specific cleavage band (∼ 55 kDa), similar to the control mCherry-SctQ (lane 6). Detected proteins and expected sizes: 1, Zdk1-mCherry-SctQ, 67.8 kDa; 2, Zdk1-SctQ, 40.8 kDa; 3, SspB_Nano-mCherry-SctQ, 73.7 kDa; SspB_Nano-SctQ, 46.7 kDa; 5, WT SctQ, 34.4 kDa; 6, mCherry-SctQ, 62.6 kDa. (D) Western blot anti-mCherry of mCherry-labeled anchor and bait combinations of both LITESEC systems. Detected proteins and expected sizes: A, Zdk1-mCherry-YscQ and TMH-FLAG-mCherry-LOV2, 67,8 and 47,6 kDa; B, SspB_Nano-mCherry-YscQ and TMH-FLAG-mCherry-iLID, 73,7 kDa and 48,7 kDa.

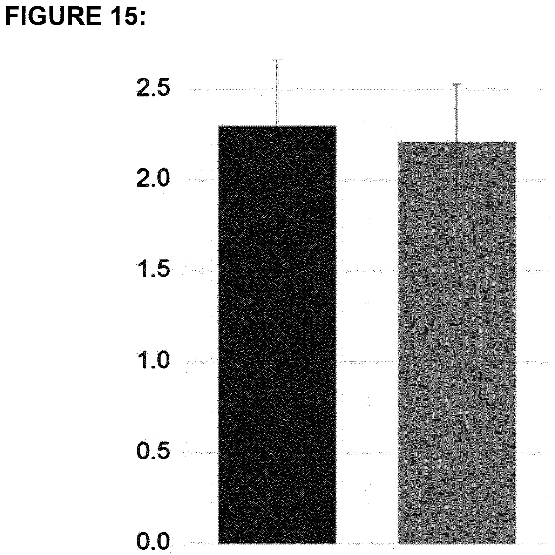

**Figure 15** shows that illumination with blue light in the used intensity does not significantly influence growth and division of Y. *enterocolitica.* Average optical density at 600 nm of wild-type cultures in secreting conditions after 180 min in dark conditions (grey, left) or light conditions (blue, right) as used in the optogenetics experiments. n=3, p=0.77.

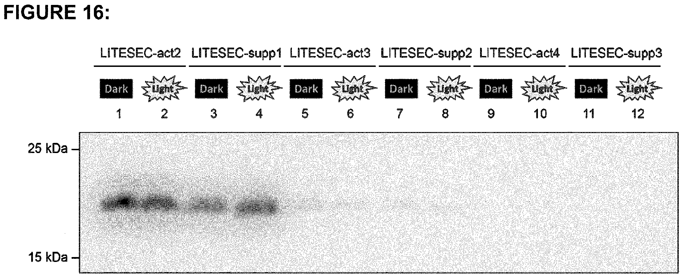

**Figure 16** shows the expression levels of membrane anchor proteins. Western blot anti-FLAG of total cellular protein from 1.5^{∗}10⁹ bacteria in the indicated strains (see Table 3 for strain details).

### DETAILED DESCRIPTION OF THE INVENTION

The present invention is based on the surprising observation, that by anchoring a member of a light-dependent protein binding pair, for example to the cytoplasmic membrane, the activity of a protein of interest, which causes or modulates a cellular function of the bacterial host cell, and which is coupled to the other member of the light-regulated protein binding pair, can be switched on and off by light-induced cleavage and reformation of the light-dependent protein binding pair.

Thus, in a first aspect, the present invention relates to recombinant gram-negative bacterium comprising a type III secretion system, wherein said type III secretion system is light-dependent.

In the context of the present invention, the term "type III secretion system " refers to the bacterial type III secretion system (T3SS) injectisome. The injectisome is a bacterial nanomachine comprising a protein complex capable of translocating proteins, so-called effectors, into eukaryotic host cells in a one-step export mechanism across the bacterial and eukaryotic membranes (Deng et al, 2017; Wagner et al, 2018) (Fig. 1). The core components of the injectisome, called type III secretion system (T3SS), are shared with the bacterial flagellum (Diepold & Armitage, 2015). The injectisome is a large transmembrane complex that bridges the space to the target cell with a hollow extracellular needle and consists of (i) an extracellular needle formed by helical polymerization of a small protein and terminated by a pentameric tip structure, (ii) a series of membrane rings that span both bacterial membranes and embed (iii) the export apparatus, formed by five highly conserved hydrophobic proteins thought to gate the export process, and (iv) a set of essential cytosolic components, also termed "sorting platform", which cooperate in substrate selection and export. The set of essential cytosolic T3SS components form a highly dynamic interface, in which the components permanently exchange between the injectisome and the cytosol (Diepold et al, 2015 and Diepold et al., 2017).

The injectisome is an essential virulence factor for many pathogenic Gram-negative bacteria, including *Salmonella, Shigella,* pathogenic *Escherichia coli,* and *Yersinia.* It is usually assembled upon entry into a host organism, but remains inactive until contact to a host cell has been established. At this point, two translocator proteins exported by the T3SS form a pore in the host membrane, and a pool of so-called T3SS effector proteins is translocated into the host cell at rates of up to several hundred effectors per second (Schlumberger et al, 2005; Enninga et al, 2005; Mills et al, 2008).

As a machinery evolved to efficiently translocate proteins into eukaryotic cells, the T3SS has been successfully used to deliver protein cargo into various host cells for different purposes such as vaccination, immunotherapy, and gene editing (reviewed in Bai et al, 2018). An N-terminal secretion signal as short as 15 amino acids marks cargo proteins for delivery by the T3SS (Michiels et al, 1990; Sory et al, 1995). Within the bacteria, many native cargo proteins (effectors) are additionally bound by chaperones that stabilize the cargo and enhance export (Wattiau & Cornelis, 1993; Gauthier & Finlay, 2003).

Export through the T3SS is fast and efficient: more than 10⁶ effectors can be translocated into a single host cell at rates of several hundred effectors per injectisome and second (Schlumberger et al, 2005; Enninga et al, 2005; Ittig et al, 2015). While the size and folding of the cargo proteins can influence translocation rates, and very large or stably folded proteins (such as GFP or dihydrofolate reductase) are exported at a lower rate, most cargo proteins, including proteins with molecular weights above 60 kDa, can be exported by the T3SS (Jacobi et al, 1998; Ittig et al, 2015). The amount of protein translocation into host cells can be titrated by changing the multiplicity of infection (ratio of bacteria and host cells). Within the host, the T3SS secretion signal can be cleaved off by site-specific proteases or cleavage at the C-terminus of a ubiquitin domain by the native host cell machinery (in secretion signal-ubiquitin-cargo fusions), and subcellular localization can be influenced using nanobodies co-translocated by the T3SS (Blanco-Toribio et al, 2010; Ittig et al, 2015). Taken together, these properties make the T3SS an efficient, versatile and well-controllable tool for protein delivery into eukaryotic cells.

However, T3SS inject effector proteins into host cells as soon as they are in contact (Pettersson et al, 1996). Lack of target specificity is therefore a main obstacle in the further development and application of that method (Walker et al, 2017; Felgner et al, 2017).

In the context of the present invention, the term "light-dependent" indicates that the function or feature of, or present in, the recombinant bacterium that is light-dependent, such as light-dependent protein binding or a light-dependent type III secretion system, is influenced by the presence or absence of light of a particular wavelength or wavelength spectrum. In particular, the term refers to situations, where the presence or absence of light of a particular wavelength or wavelength spectrum changes said function or feature from an "on" state to an "off" state or vice versa, such as from binding of a protein pair to non-binding, or from a light-dependent type III secretion system being active to being inactive. In particular embodiments, the term "light-dependent" refers to a function or feature that is not present in that light-dependent form in the wild-type bacterium that is the basis for the generation of the recombinant bacterium according to the present invention.

In particular embodiments, said recombinant gram-negative bacterium expresses at least one recombinant protein comprising (i) a cargo protein to be secreted by said type III secretion system and (ii) a secretion signal of said type III secretion system.

In the context of the present invention, the term "at least one recombinant protein" means that embodiments are included, wherein said recombinant gram-negative bacterium comprises one recombinant protein comprising a cargo protein that should be translocated, but that embodiments are included as well, where two or more recombinant proteins are present, each comprising such a cargo protein.

In particular embodiments, said recombinant gram-negative bacterium comprises an optogenetic interaction switch.

In particular embodiments, said optogenetic interaction switch comprises a first and a second fusion protein, which specifically bind to each other in a light-dependent way.

In particular embodiments, said recombinant gram-negative bacterium expresses (a) a first fusion protein comprising (aa) a cytosolic component of said type III secretion system, and (ab) a first component of said optogenetic interaction switch, and (b) a second fusion protein comprising (ba) an inner membrane anchor protein and (bb) a second component of said optogenetic interaction switch, wherein said first component of said optogenetic interaction switch and said second component of said optogenetic interaction switch specifically bind to each other in a light-dependent way.

In the context of the present invention, the term "specifically bind to" refers to measurable and reproducible interactions such as binding between two proteins such as a protein of interest and its cognate binding partner, which is determinative of the presence of the protein of interest in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. In its most general form (and when no defined reference is mentioned), "specific binding" is referring to the ability of the a protein of interest to discriminate between the cognate binding partner and an unrelated molecule, as determined, for example, in accordance with a specificity assay methods known in the art. Such methods comprise, but are not limited to Western blots, ELISA, RIA, ECL, IRMA, SPR (Surface plasmon resonance) tests and peptide scans. For example, a standard ELISA assay can be carried out. The scoring may be carried out by standard colour development (e.g. secondary antibody with horseradish peroxide and tetramethyl benzidine with hydrogen peroxide). The reaction in certain wells is scored by the optical density, for example, at 450 nm. Typical background (= negative reaction) may be about 0.1 OD; typical positive reaction may be about 1 OD. This means the ratio between a positive and a negative score can be 10-fold or higher. In a further example, an SPR assay can be carried out, wherein at least 10-fold, preferably at least 100-fold difference between a background and signal indicates on specific binding. Typically, determination of binding specificity is performed by using not a single reference molecule, but a set of about three to five unrelated molecules, such as milk powder, transferrin or the like. The first component of the optogenetic interaction switch of the present invention and said second component of said optogenetic interaction switch are able to specifically bind to each other under a first light condition, so that the predominant part of said first fusion protein present in said recombinant gram-negative bacterium is bound to said second fusion protein by way of the specific interaction of said first and said second component of said type III secretion system, whereas under a second light condition, the predominant part of said first fusion protein is present in free form in said recombinant gram-negative bacterium. In particular embodiments, the ratio of free first fusion protein to first fusion protein bound to said second fusion protein changes at least 5-fold, particularly at least 10-fold, more particularly at least 20-fold between the first and second light condition.

In particular embodiments, said membrane anchor is derived from the *E. coli* TatA transmembrane protein, particularly a membrane anchor comprising the the N-terminal part of TatA comprising an insertion of a Valine and a Leucine residue (see bold residues), particularly comprising the sequence MGGISIWQLLIIAVIVVLL**VL**FGTKKLGS.

In particular embodiments, said first fusion protein is expressed from a first nucleic acid sequence operably linked to first expression control sequences, and said second fusion protein is expressed from a second nucleic acid sequence operably linked to second expression control sequences, wherein expression of said first fusion protein is lower than expression of said second fusion protein, particularly lower by a factor of at least two, more particularly lower by a factor of at least five.

In particular embodiments, said cytosolic component is a component of said type III secretion system with native low expression and/or low stoichiometry, and/or wherein said first nucleic acid sequence is either expressed from an inducible promoter or replaces the native nucleic acid sequence encoding said cytosolic component on the virulence plasmid or in the virulence region on the bacterial genome.

In the context of the present invention, the term "virulence plasmid" refers to a plasmid of pathogenic bacteria that encodes factors responsible and required for the pathogenic activity, and the term "virulence region" relates to a corresponding region of the genome of bacteria that have integrated the virulence factors into their genome. In the case of *Yersinia enterocolitica* as an *example of a* gram-negative bacterium comprising a type III secretion system, the virulence plasmid termed pYV comprises the *ysc* and *lcr* genes, which are essential for delivery of additional plasmid-borne anti-host factors collectively referred to as Yops (*Yersinia* outer proteins). In particular embodiments, said recombinant gram-negative bacterium does not comprise a cargo protein expressed by the wild-type form of said gram-negative bacterium. Thus, while it is not excluded that said recombinant gram-negative bacterium comprises both wild type cargo proteins, i. e. cargo proteins that are translocated by said type III secretion system in a wild type gram-negative bacterium, and a recombinant fusion protein comprising a protein of interest fused to a secretion signal of said type III secretion system, it is particularly advantageous that no such wild type cargo protein is present that could compete with said recombinant fusion protein for translocation by said type III secretion system.

In particular embodiments, said recombinant gram-negative bacterium does not comprise a non-recombinant protein comprising a secretion signal of said type III secretion system.

In particular embodiments, said recombinant gram-negative bacterium is from a species selected from the group of *Yersinia, Pseudomonas, Escherichia coli, Salmonella, Shigella, Vibrio, Burkholderia, Chlamydia*, *Erwinia, Ralstonia, Xanthomonas,* and *Rhizobium.*

In particular embodiments, said recombinant gram-negative bacterium is from a species selected from *Yersinia, Pseudomonas, Escherichia coli,* and *Salmonella,* particularly selected from *Yersinia* and *Pseudomonas.*

In particular embodiments, said recombinant gram-negative bacterium is selected from *Yersinia enterocolitica and Pseudomonas aeruginosa.*

In particular embodiments, said recombinant gram-negative bacterium is from *Yersinia enterocolitica.*

The gram-negative, rod-shaped, facultative anaerobe enterobacterium Y. *enterocolitica* is able to colonize, invade and multiply in host tissues and cause intestine diseases that are commonly called yersiniosis. Essential for virulence is the translocation of six Yop (Yersinia outer protein) effector proteins into phagocytes, which prevent phagocytosis and block pro-inflammatory signaling (Cornelis, 2002).

In particular such embodiments, the six main virulence effectors of *Yersinia enterocolitica* have been deleted.

In the context of the present invention, the phrase "six main virulence effectors" refers to the six Yop (Yersinia outer protein) effector proteins.

In particular embodiments, said recombinant gram-negative bacterium is from strain IML421 asd.

In the context of the present invention, the term "strain IML421 asd" refers to the strain IML421asd (ΔHOPEMTasd) as described by Kudryashev et al, 2013, where the six main virulence effectors have been deleted.

In particular embodiments, said cytosolic component is selected from SctK, SctL, SctQ, and SctN.

In the context of the present invention, the terms "SctK", "SctL", "SctQ", and "SctN" refer to the four soluble cytosolic components of the T3SS (SctK, L, Q, N, previously called YscK, L, Q, N) in *Yersinia enterocolitica,* which interact with each other, and form a complex at the proximal interface of the injectisome (Morita-ishihara et al, 2005; Johnson & Blocker, 2008; Biemans-Oldehinkel et al, 2011; Diepold et al, 2017; Hu et al, 2017; Lara-Tejero et al, 2019) (Fig. 2A). All four proteins are needed for protein export under normal conditions, and require each other's presence for assembly at the injectisome. As these proteins were found to interact with export substrates, effectors and their chaperones, in a graded affinity that matches the export order, they were termed "sorting platform" (Lara-Tejero et al, 2011). It was recently discovered that the sorting platform proteins of the Y. *enterocolitica* T3SS constantly exchange between the injectisome and a cytosolic pool (Fig. 2BC), and that this exchange is linked to protein secretion by the T3SS (Fig. 2D) (Diepold et al, 2015; Diepold et al, 2017). The presence of an unbound cytosolic sorting platform pool has recently been confirmed for the Salmonella SPI-1 T3SS (Zhang et al, 2017), suggesting that sorting platform dynamics is a common feature of all T3SS. The dynamic exchange of these essential T3SS components opens up a completely new way to regulate the activity of the T3SS via specific sequestration and release of a cytosolic T3SS component.

In particular embodiments, said cytosolic component is SctQ.

In particular embodiments, the type III secretion system is functionally inactive in the absence of light of a particular wavelength, and can be functionally activated by illumination with light of said wavelength.

In particular such embodiments, said optogenetic interaction switch is the LOV switch or an optogenetic interaction switch derived therefrom.

In the context of the present invention, the term "LOV switch" refers to the LOVTRAP system (LOV), which consists of the two interacting proteins LOV2 (a photo sensor domain from *Avena sativa* phototropin 1) (anchor) and Zdk1 (Z subunit of the protein A) (bait). These proteins are usually bound to each other in the dark. After irradiation with blue light (∼ 480 nm wavelength) LOV2 undergoes a conformational change and Zdk1 is released. Wang and coworkers have established several point mutations of the LOV2 binding domain which modulate the binding affinity and dissociation rate. In the present application, the wild type combination, which has a return rate of about 100 s (Wang et al, 2016) (Fig. 3), has been chosen.

In the context of the present invention, the term "optogenetic interaction switch derived therefrom" refers to a variant of the optogenetic interaction switch being referred to. In the case of the wild type LOV switch as disclosed in Wang et al., 2016, the optogenetic switches derived therefrom include length variants of Zdk1 and/or LOV2, or point mutations, such as the V416L point mutant of LOV2.

In particular such embodiments, said first component of said optogenetic interaction switch is Zdk1, particularly Zdk1 according to Addgene No. 81010, and said second component of said optogenetic interaction switch is LOV2 particularly LOV2 according to Addgene No. 81041, or the V416L point mutation thereof.

### Bait: Zdk1

- generated by mRNA display screening of a library derived from the Z subunit of protein A
- Addgene code: p81010
- Sequence of bait Zdk1 -YscQ *(Zdk1 sequence in italics,* **YscQ sequence in bold,** linker underlined):

MSLRSGAGVDNKFNKEKTRAGAEIHSLPNLNVEQKFAFIVSLFDDPSQSANLLA
EAKKLNDAQAPKGGSELGGSGGSGGSLLTLPQAKLSELSLRQRLSHYRQNYL
WEEGKLELTVSEPPSSLNCILQLQWKGTHFTLYCFGDDLANWLTPDLLGAPFS
TLPKELQLALLERQTVFLPKLVCNDIATASLSVTQPLLSLRLSRDNAHISFWLT
SAEALFALLPARPNSERIPLPILLSLRWHKVYLTLDEVDSLRLGDVLLAPEGSG
PNSPVLAYVGENPWGYFQLQSNKLEFIGMSHESDELNPKPLTDLNQLPVQVSF
EVGRQILDWHTLTSLEPGSLIDLTTPVDGEVRLLANGRLLGHGRLVEIQGRLGV
RIERLTEVTIS

### Anchor: LOV2 (V416L)

- Photosensor domain from *Avena sativa* phototropin 1
- Mutation V416L is said to lower binding affinity
- Addgene code: p81041
- Sequence of anchor (membrane anchor-FLAG tag in bold, LOV2 sequence in italics mutation V416L underlined):

MGGISIWQLLIIAVIVVLLVLFGTKKLGSDYKDDDDKGGAGGSLATTLERIEKNF
LITDPRLPDNPIIFASDSFLQLTEYSREEILGRNCRFLQGPETDRATVRKIRDAIDN
QTEVTVQLINYTKSGKKFWNLFHLQPMRDQKGDVQYFIGVQLDGTEHVRDAAE
REGVMLIKKTAENIDEAAKEL

In particular embodiments, the type III secretion system is functionally inactive in the presence of light of a particular wavelength, and can be functionally activated by removing illumination with light of said wavelength.

In particular such embodiments, said optogenetic interaction switch is the Magnet switch, or an optogenetic interaction switch derived therefrom.

In the context of the present invention, the term "Magnet switch" refers to a system, which consists of two engineered photoreceptors VVD, called Magnets, which were derived from the filamentous fungus Neurospora crassa. These Magnet proteins bind to each other upon irradiation with blue light and dissociate to an "off-state" in the dark. Several mutations and combinations were designed (Kawano et al, 2015), which allows dissociation rates from seconds to hours. We chose the combination of pMAGFast2(3x) (anchor) and nMAGHigh1 (bait), which have a dissociation rate of 40-60 s (Kawano et al, 2015)

In particular embodiments, said first component of said optogenetic interaction switch is nMAGHigh1, particularly nMAGHigh1 according to Addgene No. 67300, and said second component of said optogenetic interaction switch is pMAGFast2(3x), particularly pMAGFast2(3x)* according to Addgene No. 67297, or a variant of pMAGFast2(3x)* with two instead of three repeats of the domain.

In other particular embodiments, said optogenetic interaction switch is the iLID switch, or an optogenetic interaction switch derived therefrom.

In the context of the present invention, the term "iLID switch" refers to a system that consists of two interacting proteins: iLID (anchor), which is derived from an LOV2 domain from Avena sativa phototropin 1 and a binding partner, in the present case SspB_Nano (bait). This combination was chosen because of its fast recovery half-time of 90-180 s. The iLID system has a low binding affinity in the dark and a high affinity upon irradiation with blue light (Guntas et al, 2015; Zimmerman et al, 2016).

In particular such embodiments, said first component of said optogenetic interaction switch is SspB, particularly SspB_Nano according to Addgene No. 60409, and said second component of said optogenetic interaction switch is iLID particularly iLID according to Addgene No. 60408, or the C530M point mutation thereof.

### Anchor: iLID (C530M)

- derived from an LOV2 domain from *Avena sativa* phototropin 1
- Addgene code: p60408
- Sequence: TMH-FLAG-*iLID* (iLID sequence in italics:

MGGISIWQLLIIAVIVVLLVLFGTKKLGSDYKDDDDKGGAGGSGEFLATTLERIEK
NFVITDPRLPDNPIIFASDSFLQLTEYSREEILGRNCRFLQGPETDRATVRKIRDAI

DNQTEVTVQLINYTKSGKKFWNVFHLQPMRDYKGDVQYFIGVQLDGTERLHGA
AEREAVMLIKKTAFQIAEAANDENYF

### Bait: SspB Nano

- Addgene code: p60409
   Sequence of bait *(SspB_Nano sequence in italics,* **YscQ sequence in bold,** linker underlined):

MSLRSGAGSSPKRPKLLREYYDWLVDNSFTPYLVVDATYLGVNVPVEYVKDGQ
IVLNLSASATGNLQLTNDFIQFNARFKGVSRELYIPMGAALAIYARENGDGVMFE
PEEIYDELNIGGAGELGGSGGSGGSLLTLPQAKLSELSLRQRLSHYRQNYLWE
EGKLELTVSEPPSSLNCILQLQWKGTHFTLYCFGDDLANWLTPDLLGAPFSTL
PKELQLALLERQTVFLPKLVCNDIATASLSVTQPLLSLRLSRDNAHISFWLTSA
EALFALLPARPNSERIPLPILLSLRWHKVYLTLDEVDSLRLGDVLLAPEGSGPN
SPVLAYVGENPWGYFQLQSNKLEFIGMSHESDELNPKPLTDLNQLPVQVSFEV
GRQILDWHTLTSLEPGSLIDLTTPVDGEVRLLANGRLLGHGRLVEIQGRLGVRI
ERLTEVTIS

In other embodiments of the recombinant gram-negative bacterium of the present invention, the type III secretion system is functionally inactive in the presence of light of a particular first wavelength, and is functionally active in the presence of light of a particular second wavelength.

In particular such embodiments, said optogenetic interaction switch is the Phy-PIF switch, or an optogenetic interaction switch derived therefrom.

In particular such embodiments, said first component of said optogenetic interaction switch is a fragment of a phytochrome interaction factor protein (PIF), and said second component of said optogenetic interaction switch is a Phy variant.

In particular such embodiments, said PIF fragment is a fragment of A. thaliana PIF3 protein, and said second component of said optogenetic interaction switch is a Phy variant, particularly a Phy variant consisting of residues 1-621 of the *A. thaliana* PhyB protein.

In particular other such embodiments, said PIF fragment is a fragment of A. thaliana PIF6 protein, particularly a PIF fragment consisting of residues 1-100 of A. thaliana PIF6 protein, and said second component of said optogenetic interaction switch is a Phy variant, particularly a Phy variant consisting of residues 1-901 of the *A. thaliana* PhyB protein.

In particular such embodiments, said Phy variant is fused N-terminally of said inner membrane anchor protein, particularly linked by the linker EFDSAGSAGSAGGSS.

In these embodiments, a membrane-permeable small molecule chromophore is needed for light-induced interaction. In particular embodiments, said recombinant gram-negative bacterium comprises phycocyanobilin **(PCB).** In particular such embodiments, PCB is present in or added to the culture medium. In other such embodiments, PCB synthesis is integrated inside said recombinant gram-negative bacterium, particularly by a two-plasmid system comprising a first plasmid expressing an apophytochrome, and a second plasmid expressing a dual gene operon containing a heme oxygenase and a bilin reductase.

In these embodiments of an optogenetic switch, exposure to light of a wavelength of 650 nm induces association of PIF and Phy, while exposure to light of a wavelength of 750 nm induces dissociation of PIF from Phy.

In a second aspect, the present invention relates to a method for modifying the translocation of one or more cargo proteins from a recombinant gram-negative bacterium, comprising the steps of (i) culturing a recombinant gram-negative bacterium comprising a light-dependent type III secretion system of the present invention under a first light condition, and (ii) culturing said recombinant gram-negative bacterium under a second light condition, wherein the change from said first light condition to said second light condition modifies the translocation activity of said light-dependent type III secretion system

In particular embodiments, said translocation activity is secretion of said one or more cargo proteins into the culture medium.

In other particular embodiments, said translocation activity is transfer of said one or more cargo proteins into a eukaryotic host cell.

In particular embodiments, said recombinant gram-negative bacterium expresses (a) a first fusion protein comprising (aa) a secretion signal, and (ab) a first component of said optogenetic interaction switch, and (ac) a cargo protein to be translocated by the type III secretion system, and (b) a second fusion protein comprising (ba) an inner membrane anchor protein and (bb) a second component of said optogenetic interaction switch, wherein said first component of said optogenetic interaction switch and said second component of said optogenetic interaction switch specifically bind to each other in a light-dependent way.

In a third aspect, the present invention relates to a recombinant bacterium wherein said recombinant bacterium comprises an optogenetic interaction switch to control one or more cellular functions.

In particular embodiments, said optogenetic interaction switch comprises a first and a second fusion protein, which specifically bind to each other in a light-dependent way.

In particular embodiments, said recombinant bacterium expresses (a) a first fusion protein comprising (aa) a first component of said optogenetic interaction switch, and (ab) the protein of interest whose one or more functions should be controlled in a light-dependent way, and (b) a second fusion protein comprising (ba) an anchor protein, wherein said anchor protein fixes said first second fusion protein to an organelle of said recombinant bacterium, particularly to the inner membrane, and (bb) a second component of said optogenetic interaction switch, wherein said first component of said optogenetic interaction switch and said second component of said optogenetic interaction switch specifically bind to each other in a light-dependent way.

In the context of the present invention, the term "organelle" refers in general to structural subunits of bacteria, including the outer cell wall, the cytoplasmic membrane, additional intracellular membranes, the bacterial chromosome, plasmids, any cytoskeleton structures, nutrient storage structures, and microcompartments. In particular, the term "organelle" refers to the cytoplasmic (or plasma) membrane.

In particular embodiments, one or more functions of said protein of interest within the bacterium are inhibited by light-dependent binding of said anchor protein to said organelle, particularly to the inner membrane, particularly wherein said protein of interest functions within the cytosol, or has an intermediate cytosolic state.

In particular embodiments, the one or more functions of said protein of interest are inhibited by light-dependent binding of said anchor protein to said organelle, particularly to the inner membrane, because said protein of interest cannot interact with any of its native target, or fulfil its native role, when in proximity to said membrane anchor.

In other particular embodiments, where the function of the target protein is inhibited by light-dependent binding to the membrane anchor, because a binding interface of said protein of interest that is required for any of the native functions of said protein of interest is inaccessible, when said anchor protein is bound to said organelle.

In a fourth aspect, the present invention relates to method for modifying at least one cellular function of a recombinant bacterium, comprising the steps of (i) culturing a recombinant bacterium comprising an optogenetic interaction switch of the third aspect of the present invention under a first light condition, and (ii) culturing said recombinant bacterium under a second light condition, wherein the change from said first light condition to said second light condition modifies at least one cellular function.

### EXAMPLES:

### LITESEC-T3SS - Protein secretion and translocation into eukaryotic cells with high spatial and temporal resolution by light-controlled activation of the bacterial type III secretion system

### Abstract

In this study, we apply T3SS dynamics to control protein secretion and translocation by the T3SS, by coupling these dynamic proteins with optogenetic interaction switches featuring a membrane-bound anchor domain. Initially, we screened and optimized several optogenetic systems for a proof of principle for the establishment of optogenetic interaction control in prokaryotes. Next, we incorporated the essential dynamic cytosolic T3SS component SctQ into the most suitable systems, which allows controlling the availability of this component, and in consequence secretion of effector proteins through the T3SS by light. Different versions of our resulting LITESEC-T3SS (**L**ight-**i**nduced **s**ecretion of **e**ffectors through **s**equestration of **e**ndogenous **c**omponents of the **T3SS)** system achieve fast and specific temporal extraction or release of SctQ. Strikingly, *in vitro* secretion assays confirmed that these systems allow to both activate or block secretion of effector proteins through the T3SS by blue light, permitting spatially and temporally resolved protein translocation into host cells.

### Function and regulation of the T3SS in Yersinia enterocolitica

The gram-negative, rod-shaped, facultative anaerobe enterobacterium Y. *enterocolitica* is able to colonize, invade and multiply in host tissues and cause intestine diseases that are commonly called yersiniosis. Essential for virulence is the translocation of six Yop (*Yersinia* outer protein) effector proteins into phagocytes, which prevent phagocytosis and block pro-inflammatory signaling (Cornelis, 2002). In this study, we use the strain IML421asd (ΔHOPEMTasd) (Kudryashev et al, 2013), where the six main virulence effectors have been deleted. Formation of the injectisome is often induced by temperature. In *Yersinia* sp., incubation at 37°C, the host body temperature, leads to expression of the main T3SS transcription factor VirF/LcrF by the dissociation of the repressor YmoA, which blocks its transcription at lower temperatures (Lambert de Rouvroit et al, 1992). This activates the expression of the T3SS genes on the pYV virulence plasmid, and assembly of injectisomes. Secretion of effector proteins is then triggered by host cell contact or low Ca²⁺ in the medium (Cornelis, 2006).

### Dynamics of the cytosolic components of the T3SS and its link to effector secretion

In this work, three different optogenetic interaction switches were used to sequester cytosolic proteins to the bacterial inner membrane (IM) (Table 1): (i) the LOVTRAP system (LOV), (ii) the Magnet system, and (iii) the iLID system.

**Table 1: Overview of optogenetic systems**

| Optogenetic systems that were investigated in this work with properties and application in LITESEC-T3SS. *, due to recombination events during cloning, the used anchor for the Magnet-based sequestration system only contained two consecutive copies of the pMAGFast2 protein, and is therefore expected to display a slightly lower dissociation rate, compared to the original 3x version (Kawano et al, 2015). | | | | | |
|---|---|---|---|---|---|
| **System** | **System class and properties** | **Used *anchor* and bait proteins** | **Dissociation rate after activation** | **Ref.** | **Application in LITESEC-T3SS** |
| **LOV** | Light-released Dark = bound state | *LOV2* Zdk1 | ∼ 100s | Wang *et al*, 2016 | Release of bait protein by blue light → activation of secretion |
| | Light = unbound state | | | | |
| **Magnet** | Dark-released Dark = unbound state | *pMAGFast2(2x)* nMAGHigh1 | ∼ 40-60 s | Kawano *et al*, 2015 | Tethering of bait protein by blue light → suppression of secretion |
| | Light = bound state | | | | |
| **iLID** | Dark-released Dark = unbound state | *iLID* SspB Nano | ∼ 90-180 s | Guntas *et al*, 2015 | Tethering of bait protein by blue light → suppression of secretion |
| | Light = bound state | | | | |

### Aim of this study

By combining a light-induced protein interaction domain with an essential dynamic type III secretion system (T3SS) component, we aim to control the availability of the component, and in consequence T3SS-based protein translocation into host cells, by light. The resulting system allows spatially and temporally resolved protein translocation into host cells.

Optogenetic systems were mainly established and studied in eukaryotic cells (Mukherjee et al, 2017; Wang et al, 2016; Zimmerman et al, 2016; Kawano et al, 2015). Bacteria are less compartmentalized than eukaryotic cells. We therefore designed a system where one interaction partner of the interaction switch was tethered to the bacterial inner membrane (IM). As a proof of principle, we assessed the effect of illumination on the different switches by light microscopy, using a fluorescently labeled bait protein. This allowed to optimize the systems by adjusting expression levels of anchor and bait proteins, and intensity and duration of illumination. Having demonstrated that these optogenetic sequestration systems can be used in bacteria, we fused an essential Y. *enterocolitica* cytosolic T3SS component to the respective bait protein to control its availability and, in consequence, secretion of effector proteins through the T3SS by light. The successful development of this system enables widespread opportunities for using the T3SS as a specific and time-controlled tool to deliver proteins of interest into eukaryotic cells (Ittig et al, 2015; Bai et al, 2018).

### Results

### Establishment and optimization of optogenetic sequestration systems in Y. enterocolitica

### Design and functionality of light-controlled protein sequestration systems

Optogenetic systems were mainly established and studied in eukaryotic cells (Mukherjee et al, 2017), most optogenetic applications have not been used or characterized in bacteria so far. For that reason, the first step of our research was to establish optogenetic sequestration systems in Y. *enterocolitica* to act as proofs of principle and to allow the optimization of the resulting strains for the application in the LITESEC systems. For the sequestration systems, the larger optogenetic interaction partner of all three underlying optogenetic interaction switches was anchored to the inner membrane (anchor). This was achieved by adding the N-terminal transmembrane helix (TMH) of a well-characterized transmembrane protein, *Escherichia coli* TatA, which was extended by two amino acids for more stable insertion in the IM, and connected with the interaction partner by a linker containing short Glycine-rich stretches for flexibility and a FLAG tag for detection (see material and methods for details). The smaller interaction partner was fused to a fluorescent protein for the proof of principle, or the dynamic T3SS component for the final LITESEC constructs (bait). This strategy increased the chance of obtaining functional fusion proteins.

To characterize the resulting optogenetic sequestration systems in living bacteria, we visualized fusions of fluorescent proteins to either the membrane anchor, or the cytosolic bait (Fig. 4A). To allow complete binding of the bait to the membrane anchor, we aimed at high expression levels of the anchor, and expressed the membrane anchor constructs from the inducible medium-high copy expression vector pBAD-His/B. The cytosolic bait fusions were expressed from a compatible low copy vector, pACYC184. Table 2 displays the constructs for the three optogenetic systems LOV, Magnet and iLID.

**Table 2:**

| Optogenetic constructs for membrane sequestration assay Constructs of the interaction partners used for the membrane sequestration assay, and their domains. TMH, extended transmembrane helix (see material and methods for details). | | | |
|---|---|---|---|
| Optogenetic system | Plasmid name | Domains of expressed protein | Role of expressed protein |
| **LOV** | *pAD608* | *TMH-FLAG-LOV2* | *Anchor* |
| | *pFL 100* | *TMH-FLAG-mCherry-LOV2* | |
| | pFL101 | Zdk1-EGFP | Bait |
| | pFL104 | Zdk1-mCherry | |
| **Magnet** | *pAD614* | *TMH-FLAG-pMAGFast2* | *Anchor* |
| | *pFL102* | *TMH-FLA G-mCherry-* | |
| | pFL103 | *pMAGFast2* | Bait |
| | pFL106 | nMAGHigh1-EGFP | |
| | | nMAGHigh1-mCherry | |
| **iLID** | *pFL 108* | *TMH-FLAG-iLID* | *Anchor* |
| | *pFL107* | *TMH-FLAG-mCherry-iLID* | |
| | pFL109 | SspBNano-mCherry | Bait |

To visualize the localization of the two interacting optogenetic proteins of the LOV system and the Magnet system, combinations of mCherry-labeled anchors and corresponding EGFP-labeled bait proteins were transformed into Y. *enterocolitica.* The strains were grown at ambient light and visualized with a fluorescence microscope (no pre-irradiation with blue light of ∼ 480 nm). The membrane-anchored proteins fused to mCherry showed a strict localization on the membrane and no fluorescence signal in the cytosol (Fig. 4B, top), indicating stable fusions and a functional TMH motif. In the absence of the anchor, the bait proteins were strictly cytosolic (shown for the LOV bait in Fig. 4C). In the presence of the membrane anchor, the LOV bait partially localized to the membrane, while the Magnet bait was predominantly localized in the cytosol (Fig. 4B, bottom). Taken together, these results indicate that the LOV bait protein is partially bound to the anchor within *Y. enterocolitica* at ambient light, whereas the Magnet bait is not.

### Light-dependent protein sequestration in Y. enterocolitica

Next, we tested the effect of blue light on the localization of the bait proteins in the different systems. For each tested optogenetic interaction switch, we combined a non-fluorescent anchor and fused the bait to mCherry, which has an excitation wavelength that does not overlap with the activation wavelength of the optogenetic switch systems. To exclude any effect of blue light components of ambient light on the tested samples, we incubated Y. *enterocolitica* expressing the respective protein pairs in the dark or under blue light (see material and methods for details), and fixed the cells prior to analysis at the fluorescence microscope.

The bait protein in the LOV-based sequestration system was released to the cytosol upon illumination, albeit incompletely, and still displayed partial membrane localization in light conditions (Fig 5, left). This indicates that, as expected, the interaction partners bind to each other under dark conditions and that this binding can be, at least partially, abolished under blue light. The bait protein in the Magnet-based sequestration system localized to the membrane to a low degree after under light conditions. In the dark, it remained completely cytosolic (Fig 5, center). This indicates that the binding of the two interaction partners of the Magnet system can be incompletely induced by blue light in our system. Notably, several cells of this strain showed bright polar foci in different sizes (see also Fig. 4). These might be inclusion bodies, possibly indicating low solubility of the bait protein. In the iLID-based sequestration system, the bait was mainly cytosolic in the dark, with weak membrane localization. Under blue light, the fluorescence signal changed to a predominantly membrane-bound localization (Fig 5, right). Some bacteria show brightly fluorescent polar foci, but to a lower degree than in the Magnet system. In summary, binding of the two interaction partners of the sequestration systems can be influenced by blue light in our systems.

### Expression level and stability of optogenetic fusion proteins

To investigate possible reasons for the incomplete binding or release of the bait proteins (Fig. 5), we compared the expression levels of anchor and bait proteins for the tested systems. Optogenetic interaction switches work best if the membrane anchor is expressed five to ten times higher than the bait (Wang et al, 2016). We therefore tested the stability and expression ratio of mCherry fusions to the components of all tested optogenetic systems in an anti-mCherry western blot (Fig. 6). In all tested constructs, bands of the expected size were predominant, but we detected weaker, and system-independent, bands of lower MW for all proteins. In the LOV-based system, the band intensities of anchor and bait protein are similar, which indicates an equal expression level of these two proteins (Fig. 6, lane 3). The membrane anchor protein of the Magnet-based system is expressed at a lower level than the cytosolic bait (Fig. 6, lane 6). The membrane anchored protein of the iLID-based system shows a higher expression level than the cytosolic protein (Fig. 6, lane 9).

Based on these results, we used the iLID-based sequestration system, which showed the most suitable expression ratio and a strong reaction to blue light, and the LOV-based sequestration system, which is the only light-released system, allowing to activate the T3SS in the final LITESEC system, in the next experiments.

### Interaction and recovery dynamics of the optogenetic sequestration systems

To test the dynamics of the sequestration switches in live Y. *enterocolitica,* time-lapse experiments with the iLID-based and the LOV-based sequestration systems were performed. In each case, the localization of the mCherry-bait fusion in live Y. *enterocolitica* grown in the dark was determined by fluorescence microscopy. The system was then activated by a short pulse of blue light (0.1 sec of GFP excitation light (∼ 480 nm)), and changes in bait localization were tracked over time (Fig. 7AB, Fig. 8). To quantify the change of the normalized fluorescence signal over time, line scans were performed (Fig. 7CD). For the iLID system, in the pre-activated state, the fluorescence signal of the bait-mCherry was cytosolic. After activation with blue light, the fluorescence signal partly shifted to the membrane (Fig. 7A) and returned to the cytosol within the next minutes (Fig. 7C, Fig. 8). For the LOV-based sequestration system, the fluorescence signal of the bait-mCherry was mainly membrane localized in the pre-activated state. Activation with blue light, only lead to a minor relocalization of the signal from the membranes to the cytosol (Fig. 7AC, Fig. 8).

### Optogenetic control of T3SS effector secretion

To establish light control over protein translocation activity of the T3SS, we developed two complementary systems, based on the results of the previous experiments:

### A) LITESEC-supp, a system that confers suppression of T3SS-dependent protein translocation by blue light illumination

### B) LITESEC-act, a system that confers activation of T3SS-dependent protein translocation by blue light illumination

Both systems rely on two interaction partners which we have engineered:
(i) the membrane-bound anchor proteins used in the previous experiments, fusions between an extended trans-membrane helix, a Flag peptide for detection and spacing, and the larger component of the interaction switches that performed best in the sequestration assays, iLID (LITESEC-supp) / LOV2 (LITESEC-act). As in the preliminary experiments, the resulting fusion proteins, **TMH-iLID** / **TMH-LOV2,** are expressed from a plasmid;
(ii) fusion proteins between an essential cytosolic T3SS component, SctQ, and the smaller component of the interaction switches, SspB_Nano (LITESEC-supp) / Zdk1 (LITESEC-act). The two domains of the fusion proteins are connected by a flexible Glycine-rich peptide linker that was shown to retain the functionality of SctQ fusion proteins (Diepold *et al*, 2010, 2015). The resulting fusion proteins, **SspB_Nano-SctQ** / **Zdk1-SctQ,** replace the wild-type SctQ protein on the virulence plasmid (allelic exchange of the genes, (Kaniga *et al*, 1991)).

The two proteins were co-expressed in a non-virulent Y. *enterocolitica* strain lacking its native virulence effectors to allow optogenetic (light-induced) control of protein translocation by the T3SS (Fig. 8).

For the iLID-based LITESEC-supp system, in the light, the bait protein is tethered to the membrane anchor (Fig. 8A, left), and is therefore not available for the T3SS. As SctQ is essential for the function of the T3SS (Diepold et al, 2010), protein secretion by the T3SS is prevented (Fig. 8B). In the dark, the bait protein is not bound to the membrane, and can therefore functionally interact with the T3SS, which allows protein secretion by the T3SS (Fig. 8C). Conversely, in the LOV-based LITESEC-act, the bait protein is released from the membrane upon irradiation with blue light, licensing protein secretion by the T3SS (Fig. 8).

### Expression levels and stability of LITESEC components

We confirmed that the SctQ fusion proteins used in the LITESEC system, Zdk1-SctQ and SspB_Nano-SctQ are functional (strains expressing the fusion proteins instead of wild-type SctQ secrete effectors at a normal level) (Fig. 14A). One possible reason for the incomplete relocalization of the cytosolic bait proteins in the preliminary experiments (Fig. 4, 5) is their relatively high expression level in comparison to the anchor proteins (in particular for the LOV and Magnet-based sequestration systems). We reasoned that the comparably low native expression level of T3SS components, including SctQ, might lead to a more efficient relocalization upon illumination. We therefore compared the levels of a plasmid-expressed GFP labeled bait proteins used in the preliminary experiments, Zdk1-EGFP, and the bait protein in the LITESEC strains, EGFP-SctQ, expressed from its native locus on the virulence plasmid by fluorescence microscopy. EGFP-SctQ shows a lower fluorescence intensity than Zdk1-EGFP (Fig. 14B). This indicates that the native expression level of the T3SS proteins is lower than the level of the tested cytosolic bait protein and suggests that the optogenetic sequestration will work better in these strains. We then tested the stability and expression level of the used fusion proteins in the LITESEC strains by Western blot. While bait-mCherry-SctQ fusions showed a weak degradation band at a molecular weight of 55 kDa (most likely due to internal cleavage or translation initiation in the mCherry coding sequence), the direct bait-SctQ fusions were stable (Fig. 14C), and we used these fusions in the remaining experiments. Importantly, as expected, the anchor proteins were expressed at a higher level than the bait proteins in both LITESEC systems (Fig. 14D).

### Control of protein secretion by illumination

Can we control T3SS secretion by light? We first tested the LITESEC-supp system in an *in vitro* protein secretion assay under conditions that usually lead to effector secretion (presence of 5 mM EGTA in the medium). Indeed, the light-suppressed LITESEC-supp system showed normal effector secretion when grown in the dark, but strongly reduced effector secretion when grown under blue light (λ=488 nm) (Fig. 10, lanes 1, 2). The control strain lacking the membrane anchor secreted effectors irrespective of the illumination (lanes 3, 4). Protein secretion in wild-type Y. *enterocolitica* was not influenced by the used illumination (lanes 5, 6), and the blue light had no influence on growth of *Y. enterocolitica* (Fig. 15). The secretion efficiency of the LITESEC-supp system in the dark is significantly higher than under light conditions.

### Improved functionality of the LITESEC-act system by using a mutated anchor (V416L)

We next tested the LITESEC-act1 system, where secretion is induced by blue light illumination, and detected only a very weak activation of protein export under light conditions (Fig. 11, lanes A, B). Based on the fact that secretion was wild-type-like in the absence of the membrane anchor (lane C), and the results from the earlier sequestration experiments (Fig. 5, 7), we hypothesized that bait and anchor interact too strongly in the LITESEC-act1 system.

Therefore, we constructed and tested additional versions of LITESEC-act, using the mutated anchor version V416L, which displays a weaker affinity to the bait (Wang *et al,* 2016). We introduced the mutation into the medium-high copy pBAD expression vector used for the baits in all previous experiments, as well as two low-copy vectors, pACYC184 and pMMB67EH, which we hypothesized to lead to a lower anchor/bait expression ration, and as a consequence to more efficient release of the bait and activation of T3SS secretion upon illumination. As controls, we also expressed the anchor of the LITESEC-supp1 system from the same plasmids.

We then tested the response of the resulting LITESEC systems (Table 3) to light in an *in vitro* secretion assay. In contrast to the original LITESEC-act1 strain, LITESEC-act2 showed significant induction of protein secretion in the light, compared to dark conditions (Fig. 11, lanes 1, 2). Even more strikingly, LITESEC-act3 allowed an almost complete activation of secretion upon illumination (lanes 3, 4). Both new strains retained the extremely low level of export in the dark. LITESEC-act4 showed strong activation by light, but a higher background export activity in the dark (lanes 5, 6). The LITESEC-supp2 system showed efficient secretion in the dark and strong suppression of secretion upon illumination, comparable with the LITESEC-supp1 system (lanes 7-10), while the LITESEC-supp3 system showed no activation of protein secretion under any condition (lanes 11, 12).

To determine whether the changed secretion efficiencies are indeed due to the lower expression of the anchor proteins in the new strains, we tested the expression levels by immunoblot. As expected, the anchor proteins expressed from the pBAD plasmids in the LITESEC-act2/-supp1 strains show the highest expression level (Fig. 16, lanes 1-4), the anchor proteins expressed from the pACYC184 plasmid in the LITESEC-act3/-supp2 strains display an intermediate expression level (lanes 5-8), and the pMMB67EH-based LITESEC-act4/-supp3 anchor proteins are expressed below the detection limit (lanes 9-12).

### Kinetics of light-induced T3SS activation

To test whether the function of the LITESEC system can be influenced over time, and to estimate the activation and deactivation kinetics, the LITESEC-supp1 strain and a wild-type control were incubated under secreting conditions, consecutively for 60 min under blue light, 60 min in the dark, and another 60 min under blue light. After each incubation period, the culture medium was replaced, and a sample was tested for secretion by SDS-PAGE. Secretion in LITESEC-supp1 was specifically induced in the dark, and suppressed upon illumination (Fig. 12). The WT strain continuously secreted proteins irrespective of the illumination. Based on the intensity of secretion within the 60 min periods, we estimate the both activation and suppression of secretion occur very quickly, most likely within few minutes.

**Table 3: Schematic overview of the two LITESEC systems and their optogenetic components**

| Constructs of the interaction partners used for the membrane sequestration assay, and their properties. All bait proteins are expressed from their native genetic locus. TMH, extended transmembrane helix (see material and methods for details). | | | |
|---|---|---|---|
| Optogenetic T3SS control system | Anchor *(plasmid)* | Bait | Properties |
| **LlTESEC-supp1** | TMH-FLAG-iLID *(pBAD)* | SspB_Nano-SctQ | Suppression of T3SS-based protein secretion upon illumination by membrane sequestration of essential cytosolic T3SS component |
| **LITESEC-supp2** | TMH-FLAG-iLID *(pACYC184)* | | |
| **LITESEC-supp3** | TMH-FLAG-iLID *(pMMB67EH)* | | |
| **LITESEC-act1** | TMH-FLAG-LOV2 *(pBAD)* | Zdk1-SctQ | Activation of T3SS-based protein secretion upon illumination by release of essential cytosolic T3SS component |
| **LITESEC-act2** | TMH-FLAG-LOV2_{V416L} *(pBAD)* | | |
| **LITESEC-act3** | TMH-FLAG-LOV2_{V416L} *(pACYC184)* | | |
| **LITESEC-act4** | TMH-FLAG-LOV2_{V416L} *(pMMB67EH)* | | |

### The light-dependent export of heterologous substrates by the T3SS

The T3SS-dependent export of heterologous cargo has been shown and applied for many purposes in earlier studies (Ittig *et al*, 2015; Walker *et al*, 2017; Bai *et al*, 2018). To confirm that the export of heterologous proteins can be induced in the LITESEC strains, the LITESEC-supp2 system can be combined with a standard expression vector, such as pBAD, expressing a heterologous cargo protein, expressed with a short N-terminal secretion signal (for example with YopH₁₋₁₇, the minimal secretion signal for the native Y. *enterocolitica* effector YopH, (Sory *et al*, 1995)) and a tag for detection, for example a C-terminal FLAG tag. The cargo protein can specifically be exported in the dark by the LITESEC-supp2 strain and can be detected in the medium, whereas export is light-independent in a wild-type strain.

### Discussion

### Establishing an optogenetic interaction switch in Yersinia enterocolitica

The main aim of this study was to control T3SS-based protein secretion by external light. Our system, LITESEC, is based on the sequestration of an essential dynamic T3SS component, SctQ, by an optogenetic interaction switch. As, to our knowledge, such a system had not been established in prokaryotes before, we first tested sequestration systems based on several optogenetic switches, the LOV, Magnet, and iLID systems and iLID systems (Wang et al, 2016; Kawano et al, 2015; Guntas et al, 2015), in Y. *enterocolitica.* In all cases, we expressed the larger of the two interacting proteins as a fusion to an optimized TMH, based on the N-terminal TMH of the *E. coli* TatA protein, an integral component of the Tat export system (Palmer & Berks, 2012). We tested a range of anchor / bait combinations for the different optogenetic systems, either alone or fused to fluorescent reporter proteins (Table 2), and visualized their localization in live bacteria. The membrane anchors localized exclusively to the membrane, and influenced the localization of the respective bait proteins (Fig. 4). Upon illumination with blue light, a fraction of the labeled bait proteins were bound to or released from the inner membrane (Fig. 5). Notably, sequestration or release of the bait proteins was incomplete, pointing out the need of further adjustments to the system. A possible reason is that the insufficient expression ratio between anchor and bait (Fig. 6); the anchor is optimally expressed in five- to ten-fold excess (Kawano et al, 2015) a point that we addressed in the subsequent versions of the system. Based on these initial results, we chose the LOV-based sequestration system (as a light-released system, allowing activating protein secretion in the final LITESEC-act system) and the iLID-based sequestration system (as the most efficient dark-released system, allowing to suppress protein secretion in the final LITESEC-supp system) for the next round of experiments.

### Controlling protein secretion and translocation by the T3SS with light

By fusing SctQ, an essential and dynamic cytosolic component of the T3SS (Diepold *et al,* 2015) with one of bait protein of the optogenetic sequestration systems, we established strains where the activity of the T3SS is controlled by light. We termed the resulting system LITESEC-T3SS (**L**ight-**i**nduced **s**ecretion of **e**ffectors through **s**equestration of **e**ndogenous **c**omponents of the **T3SS**). Two different LITESEC systems can be applied in opposite directions: in the LITESEC-supp system, protein export is suppressed by blue light illumination, the LITESEC-act system secretion allows to activate secretion by blue light.

Of the two original systems, the LITESEC-supp1 system, which is based on the iLID optogenetic interaction switch (Guntas *et al*, 2015), showed a significant reaction to light (Fig. 10). Expression of the membrane anchor from a constitutively active promoter on a low copy plasmid, pACYC184 (LITESEC-supp2) achieved the same activation/suppression ratio (Fig. 11), with the additional advantage that expression of the membrane anchor is constitutive.

For many applications, activation of T3SS protein export upon illumination is preferable. The LITESEC-act1 system, which is based on the LOV optogenetic interaction switch (Wang *et al*, 2016), only achieved weak activation of T3SS secretion upon illumination (Fig. 11). LITESEC-act2, which uses the V416L mutation in the anchor protein (Kawano *et al*, 2013) to decrease the affinity between anchor and bait, retained tight repression of secretion in the dark, but could be activated by light more efficiently. Even more impressively, LITESEC-act3, featuring a reduced expression level of the V416L variant of the membrane anchor, leads to an almost complete activation of T3SS protein secretion upon illumination, while retaining the tight suppression of secretion in the dark (Fig. 11). As for the LITESEC-supp2 system, expression of the membrane anchor is constitutive in this strain, and the system can be used for light-controlled export of heterologous proteins.

Reaction time and recovery dynamics of the sequestration systems are crucial for their applicability to control the function of the T3SS. Fast reaction times to blue light increase the temporal precision of T3SS activation/deactivation, whereas the recovery times influence the effect of illumination on secretion. Very fast recovery means that the system has to be continuously illuminated for a sustained effect on secretion, while very slow recovery leads to long-term activation/deactivation that is difficult to revert, and renders handling of the cultures difficult due to possible long-term effects of illumination prior to the actual experiment. In time-course experiments we could show that in the LITESEC-supp system, unbinding of the bait in the light state was almost immediate, while recovery in the dark occurred within few minutes (Fig. 7), both in line with data from eukaryotic systems (Zimmerman *et al*, 2016). For the LITESEC-act system, these properties mean that induction of protein secretion by blue light occurs within very short time, and the system remains active for several minutes. In the absence of further illumination, protein secretion is stopped within minutes, which greatly limits unwanted unspecific activation. Long-term activation can be achieved by either constant low-intensity blue light illumination, or short light pulses every few minutes.

The T3SS is a very promising tool for protein delivery into eukaryotic cells, both in cell culture and in healthcare (Ittig *et al*, 2015; Walker *et al*, 2017; Bai *et al*, 2018). However, the T3SS indiscriminately injects cargo proteins into contacting host cells (Pettersson *et al*, 1996). Lack of target specificity is therefore a main obstacle in the further development and application of this method (Walker *et al*, 2017; Felgner *et al*, 2017). Previous methods to control the activity of the T3SS relied on controlled expression of one or all components of the injectisome. For example, Song and colleagues expressed all components of the *Salmonella* SPI-1 T3SS from two inducible promoters in a clean expression system (Song *et al*, 2017), and Schulte *et al.* expressed the T3SS genes from a TetA promoter, which additionally allows the intracellular induction of the T3SS (Schulte *et al*, 2018). Besides the difficulty to specifically induce secretion in defined places *in situ,* the main drawback of these methods is the slow response (induction of expression and assembly of the T3SS take >60 min, (Diepold *et al*, 2010; Song *et al*, 2017; Schulte *et al,* 2018)), and the system remains active as long as it is in contact to a host cell, and the induced protein(s) are still present.

By using light to specifically activate the modified T3SS in bacteria at a site of choice, we have addressed this issue. The LITESEC system allows delivering proteins into host cells at a specific time and place. In addition, secretion by the LITESEC-act system is stopped within few minutes after the end of illumination with blue light, thereby further reducing unspecific activation at secondary site and side effects.

### Applications of the optogenetic switch technology:

### 1. Protein translocation into unmodified eukaryotic cells in cell culture with high temporal and spatial resolution.

Cell cultures play an important role in development, research and, increasingly, healthcare. Often, specific proteins need to be expressed in all or a subset of the cultured cells at a given time point. At the moment, this is mainly done by inducing expression of the target protein within the host cells. This method requires prior transfection of the host cells with the target gene or time-consuming creation of stable transgenic cell lines. Induction of expression itself is relatively slow, and difficult to apply to a certain subset of cells.

Our method allows translocating proteins into unmodified host cells with high specificity. Bacteria that lack their native virulence effectors, but express one or more cargo proteins with a short secretion signal, are brought into contact with host cells. The chosen subset of host cells are then subjected to darkness or blue light (which does not influence bacteria or host cells at the used intensity), which temporarily induces translocation of the cargo into the host cells within short time. An additional advantage of our method is that it directly translocates proteins into the host cell, rather than inducing the transcription of mRNA, as is the case in the current inducible transfection systems. The amount of translocated protein can be regulated by the duration of illumination/darkness, and the multiplicity of infection (ratio of bacteria / host cells) (Ittig *et al*, 2015).

### 2. Therapeutic protein delivery into diseased cells

**Specific** protein delivery into diseased cells, such as cancer cells, is one of the main targets for treating important diseases. The T3SS has been used to treat cancer cells *in vitro,* e.g. by translocating angiogenic inhibitors (Shi *et al*, 2016). A major obstacle in the further development of T3SS-based methods for clinical applications is the promiscuity of the T3SS (Walker *et al*, 2017). Most current approaches rely on localized injection of bacteria or the natural tropism of bacteria to tumorous tissue. However, bacteria applied with these methods are not restricted to the target tissue, and unspecific activation is an obstacle, especially for potentially powerful applications such as the delivery of pro-apoptotic proteins.

By using light to specifically activate the modified T3SS in bacteria at a site of choice, delivery of effector proteins could be temporarily induced at a specific time and place. This method reduces unspecific activation and side effects, allowing a highly controlled targeting of host cells. Bacteria could be applied to the patient (exploiting the natural tumor tropism of bacteria for tumor tissue in the case of cancer treatment to achieve an enrichment at the tumor site in the case of cancer), where injection of the effector protein would be triggered *in situ with* high spatial and temporal precision using light delivered with the help of endoscopes and minimally-invasive surgery techniques.

A main challenge for the *in situ* application of T3SS-based protein delivery with our LITESEC system is the wavelength of the activating light. The blue light used to control the LITESEC system does not penetrate tissue efficiently, and activation by red or far-red light would be advantageous.

### Generic description of optogenetic switch in red or far-red light.

Several such systems have been characterized (Shimizu-Sato *et al*, 2002; Reichhart *et al,* 2016; Kaberniuk *et al*, 2016), which require cofactors not usually present in bacteria. One example is the **Phy-PIF system:**
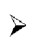 light-controllable binding interaction between two genetically encoded components:
   - a fragment of *Arabidopsis thaliana* phytochrome B (**Phy**) (anchor)
      ∘ consisting of residues 1-908 of the *A. thaliana* PhyB protein (Entrez Gene ID: 816394)
      ∘ maybe think about codon optimization (was expressed without codon optimization in yeast but codon optimization is said to increase expression ratio (Toettcher *et al*., 2011b)) - Phy-PIF recruitment is easiest to observe if Phy expression levels are high
      ∘ Phy fusion protein expression and function is particularly sensitive to linker lengths and component orientation. Phy appears to work most robustly as an N-terminal fusion component (Phy-TMH)
      ∘ Best working linker: EFDSAGSAGSAGGSS between the C-terminus of Phy and the N-terminus of downstream fusion constructs
   - and a fragment of phytochrome interaction factor 6 (**PIF**) (bait)
      ∘ consisting of residues 1-100 of *A. thaliana* PIF6 protein
      ∘ does not exhibit any preference toward N or C terminal fusions and also tolerates fusions on both termini simultaneously Source for constructs: https://www.addgene.org/browse/gene/816394/
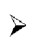 membrane-permeable small molecule chromophore, phycocyanobilin (**PCB**) is needed for light-induced interaction
   ∘ in most references, PCB was just added to the cultivation media
   ∘ PCB synthesis could also be integrated inside the cells: two-plasmid system, one expressing an apophytochrome and the other expressing a dual gene operon containing a heme oxygenase and a bilin reductase is needed (Gambetta and Lagarias, 2001)
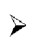 Exposure to **650 nm** induces association of PIF and Phy, while exposure to **750 nm** light induces dissociation of PIF from Phy
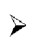 Note: two pairs are used: PhyB (1-621) + PIF3 (good for control of gene expression - more sensitive - activation also in room light) / PhyB (1-908) + PIF6 (better for protein localization control, not that sensitive - nearly no activation with normal room light) (Pathak *et al*., 2014)
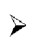 "Phy can be reversibly switched between PIF-interacting and -non-interacting states using light within seconds, and switching can be performed for hundreds of cycles without toxicity to the cell or any measurable degradation of the system's performance" - (Toettcher *et al*., 2011b)

### Material and methods

Plasmids and strains used in this study are listed in Table 6 and Table 7, respectively. Additional methods and materials are listed in "supplementary methods and materials".

### Cultivation of bacteria

All *Y. enterocolitica* strains were cultivated in BHI media (3.7 % w/v) (Brain Heart Infusion Broth - VWR Chemicals). To this medium nalidixic acid (NAL) (35 µg/ml) and 2,6-diaminopimelic acid (DAP) (60 µg/ml) were always added, because the used *Yersinia* strains are auxotrophic for DAP and have a genome encoded resistance against NAL. All *E. coli* strains were cultivated in LB media (tryptone (10 % w/v), yeast extract (5 % w/v), NaCl (10 % w/v) - CARL ROTH GmbH & CO KG (Karlsruhe, Germany)). If necessary, further antibiotics Ampicillin (Amp) (200 µg/ml) (for plates, the more stable form Carbenicillin (Carb) was used), Chloramphenicol (Cam) (25 µg/ml), Streptomycin (Sm) (50 µg/ml) depending on the integrated plasmids were added to the cultivation media. For an overnight culture, 2-5 ml of cultivation media with corresponding antibiotics were inoculated with a specific strain from the glycerol stock strain collection and were cultivated overnight at 28° C (Y. *enterocolitica*) or 37° C (*E. coli*) in a shaking incubator. For cultivation plates, 15 % w/v Agar (Becton, Dickinson and Company (New Jersey, USA)) was added to the media.

### T3SS in vitro secretion assay

From an overnight culture of strains that were planned to be examined, 100 µl (for non-secreting conditions) or 120 µl (for secreting conditions) were inoculated in corresponding media (1:50 dilution for non-secreting conditions, 1:41.67 for secreting conditions). The cultivation media contains BHI (3.7 % w/v), NAL (35 µg/ml), DAP (50 µg/ml), MgCl₂ (20 mM), glycerol (0.4 % w/v) and corresponding antibiotics. For non-secreting conditions CaCl₂ (5 mM) and for secreting conditions EGTA (5 mM) was added. The cultures were cultivated for 90 min at 28° C and then shifted to a 37° C water bath and inoculated for 2-3 h (if the strain contained an inducible plasmid, the plasmid was induced with 0.2 % w/v L-arabinose before shifting to 37° C).

### Fluorescence microscopy

For fluorescence microscopy, strains that were planned to be examined were cultivated as described above under non-secreting conditions. 2 ml of cell culture then was spun down for 4 min at 2.400 relative centrifugal force (rcf) and the cell pellet was resuspended in 400 µl of minimal media (HEPES (100 mM), (NH₄)₂SO₄ (5 mM), NaCl (100 mM), sodium glutamate (20 mM), MgCl₂ (10 mM), K₂SO₄ (5 mM), casamino acids (0.5 % w/v)) including DAP (60 µg/ml). From this culture, 2 µl was given on prepared agar slides (1.5 % w/v agarose in minimal media, heated up in microwave, 80-100 µl then put on a microscope slide with cavities (Marienfeld GmbH & Co. KG (Königshofen, Germany)) and topped with a cover slip (25 mm 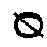 ).

On the coverslip then a drop of microscopy oil (Cargille Laboratories, Inc. (Cedar Grove, USA)) (1.514 for GFP pictures, 1.522 for mCherry pictures) was added. Samples were observed with an inverse fluorescence microscope. Unless stated differently, exposure times were 500 ms for mCherry fluorescence, using a mCherry filter set, and 200 ms for GFP fluorescence, using a GFP filter set. In dual color imaging experiments, mCherry fluorescence was excited and recorded before GFP fluorescence to minimize photo bleaching of mCherry. Per image, a z stack containing 7 to 15 frames per wavelength with a spacing of 150 nm was acquired.

### Optogenetic cell cultivation

For optogenetic experiments the strains for cell fixation or secretion assays (to determine the amount of secreted proteins) were cultivated under the presence of blue light. They were cultivated under secreting conditions as described in before but after shift to 37° C for 5 min - 1.5 h in the water bath, the cultures were cultivated at 37° C for 1 - 3 h in an optogenetic experimental setup (Fig. 13) under blue light or dark conditions. The cultures then were used for SDS-PAGE or for cell fixation and further fluorescence microscopy.

**Table 6: Strains**

| *Yersinia enterocolitica* strains that were created and/or used during this work. | | | |
|---|---|---|---|
| **Name** | **Genotype** | **Strain background** | **Comments / Reference** |
| **dHOPEMTasd** | *pYV40 yopO_{Δ2-427}yopE₂₁ yopH_{Δ1-352} yopM₂₃ yopP₂₃yopT₁₃₅ Δasd* | | (Kudryashev *et al,* 2013) |
| **AD4324** | *mCherry-SctQ* | dHOPEMTasd | (Diepold *et al,* 2015) |
| **FL02** | *Zdk1-mCherry-SctQ* | dHOPEMTasd | pFL115 x* dHOPEMTasd |
| **FL03** | *Zdk1-SctQ, mCherry-SctL* | dHOPEMTasd | pAD612 x* ADTM4521 |
| **FL04** | *SspB_ Nano-mCherry-SctQ* | dHOPEMTasd | pFL117 x* dHOPEMTasd |
| **FL05** | *SspB_Nano-SctQ, mCherry-SctL* | dHOPEMTasd | pFL118 x* ADTM4521 |
| **FL09** | *Zdk1-mCherry-SctQ, ΔSctN* | dHOPEMTasd | pAD168 x* FL02 |
| **FL10** | *SspB_Nano-mCherry-SctQ, ΔSctN* | dHOPEMTasd | pAD168 x* FL04 |
| **FL11** | *Zdk1-SctQ, mCherry-SctL*, *ΔSctN* | dHOPEMTasd | pAD168 x* FL03 |
| **FL12** | *SspB_Nano-SctQ mCherry-SctL, ΔSctN* | dHOPEMTasd | pAD168 x* FL05 |

| | | | |
|---|---|---|---|
| * x = homologous recombination between mutator-plasmid and host-strain, recombination leads to an allelic exchange of the native gene and the mutated gene. | | | |

**Table 7: Plasmids**

| Plasmids with corresponding properties that were designed and/or used in this work. | | | | | | |
|---|---|---|---|---|---|---|
| **Name (Reference)** | **Genotype** | **Restriction enzymes** | **Resist.** | **Primer used for Insert-PCR** | **Template for Insert** | **System** |
| **pFL100** | *pBAD::TMH-FLAG-(L1)-mCherry-(L2)-LOV2* | Ncol, EcoRI | Amp | AD638/704/705 | p81041** | LOV |
| **pFL101** | *pACYC184::Zdk1-(L4)-EGFP* | BamHI, SalI | Cam | AD698/699/ 700/701 | P81010**/ pAD301 | LOV |
| **pFL102** | *pBAD::TMH-FLAG-(L1)-mCherry-(L3)-pMAGFast2 (2x)* | NcoI*, EcoRI | Amp | AD638/706/ 707/708/642 | p67297**/ pAD304 | Magnet |
| **pFL103** | *pACYC184::nMAGHigh1-(L4)-EGFP* | EcoRV, BamHI | Cam | AD702/703 | p67300** | Magnet |
| **pFL104** | *pACYC184::Zdk1-(L4)-mCherry* | BamHI, SalI | Cam | AD638/699/ 721/722 | p81010**/ pAD304 | LOV |
| **pFL106** | *pACYC184::nMAGHigh1-(L4)-mCherry* | EcoRV, EagI | Cam | AD702/721/ 723/724 | p67300**/ pAD304 | Magnet |
| **pFL107** | *pBAD::TMH-FLAG-(L1)-mCherry-(L3)-iLID* | NcoI*, EcoRI | Amp | AD638/706/707/ 732/733 | p60408**/ pAD304 | iLID |
| **pFL108** | *pBAD::TMH-FLAG-(L1)-iLID* | NcoI, EcoRI | Amp | AD638/733/734 | p60408** | iLID |
| **pFL109** | *pACYC184::SspB_Nano-(L4)-mCherry* | BamHI, SalI | Cam | AD721/722/ 735/736 | p60409**/ pAD304 | iLID |
| **pFL111** | *pBAD::Zdk1-(L4)-mCherry* | BglII, EcoRI | Amp | AD759/768 | pFL104 | LOV |
| **pFL113** | *pBAD::SspB_Nano-(L4)-mCherry* | BglII, EcoRI | Amp | AD762/768 | pFL109 | iLID |
| **pFL114** | *pBAD::SspB_Nano* | Bglll, EcoRI | Amp | AD762/769 | pFL109 | iLID |
| **pFL115** | *pKNG101::Zdk1-(L4)-mCherry-SctQ* | BglII, MfeI*** | Sm | **** | pFL111 | LOV |
| **pFL117** | *pKNG101::SspB_Nano-(L4)-mCherry-SctQ* | BglII, MfeI*** | Sm | **** | pFL113 | iLID |
| **pFL118** | *pKNG101 ::SspB_Nano-SctQ* | BglII, MfeI*** | Sm | **** | pFL114 | iLID |
| **pFL126** | *pACYC184::TMH-FLAG-(L1)-LOV2 (V416L)* | BamHI***** SalI | Cam | AD921/903 | pAD610 | LOV |
| **pFL127** | *pACYC184::TMH-FLAG-(L1)-iLID* | BamHI***** SalI | Cam | AD921/904 | pFL108 | iLID |
| **pFL31******** | *pMMB67EH::TMH-FLAG-(L1)-LOV(V416L)* | BamHI***** SalI | Gm | AD921/903 | pFL126 | LOV |
| **pFL132******** | *pMMB67EH::TMH-FLAG-(L1)-iLID* | BamHI***** SalI | Gm | AD921/904 | pFL127 | iLID |
| **pAD168 (Diepold et al., 2010)** | *pKNG101::ΔSctN* | | Sm | | | |
| **pAD304 (Diepold *et al,* 2015)** | *pUC 19-mCherry* | | Amp | | | |
| **pAD608** | *pBAD::TMH-FLAG-(L1)-LOV2* | NcoI*, EcoRI | Amp | AD638/639/640 | p81041** | LOV |
| **pAD610** | *pBAD::TMH-FLAG-(L1)-LOV2(V416L)* | NcoI*, EcoRI | Amp | | | LOV |
| **pAD612** | *pKNG101::Zdk1-SctQ* | Bglll, MfeI*** | Sm | **** | pAD611 | LOV |
| **pAD614** | *pBAD::TMH-FLAG-(L1)-pMAGFast2(2x)* | NcoI*, EcoRI | Amp | AD638/641/642 | p67297** | Magnet |

| | | | | | | |
|---|---|---|---|---|---|---|
| * Insert was digested with Bsal instead of Ncol ** Addgene code *** Insert was digested with EcoRI instead of Mfel **** Insert was cut out of pre-mutator and ligated to mutator-vector ***** Insert was digested with Bglll instead of BamHI ****** pMMB67EH-MA was designed without promotor region L1 - GAGG linker L2 - GSGS linker L3 - GAGGGAGG linker L4 - GGSGGSGG linker | | | | | | |

### Supplementary methods and materials

### Plasmid construction

All plasmids that were designed and made in this work are listed in Table 4. Primer that were designed and used for PCR of the plasmid-specific inserts are listed in Table S1.

PCR products were purified by using a purification kit or by gel electrophoresis (1:6 6x loading dye (Bromphenol blue (0.25 % w/v), Xylene cyanol FF (0.25 % w/v), Glycerol (30 % w/v) in PCR reaction mix, load on an agarose gel (1 % w/v Agarose, 1x TAE buffer (TRIS-acetate (40 mM), EDTA (1 mM), pH = 8.3), EtBr (0,05 % w/v)) - settings: 135 V, 500 mA, 30 min) and following gel extraction of the band of correct size that was cut out.

Purified PCR products and corresponding vector were digested with corresponding restriction enzymes and settings (shown on NEB cloner) depending on the used enzymes (usually 1 h at 37° C and specific restriction buffer). The digested vector was treated with Antarctic Phosphatase (2 % w/v) (plus 10x phosphatase buffer - 10 % w/v) (New England Biolabs GmbH (Frankfurt am Main, Germany)) that dephosphorylates the 5' and 3' ends and impede self-religation of the vector (Rina *et al,* 2000). The digestion then was purified by gel electrophoresis and gel extraction.

The digested PCR insert and vector were then ligated in a ligation mix (total volume 15 µl) that contains H₂O (15 µl - x), digested vector (100 ng), digested insert (3:1 molar ratio to vector), "10x T4 DNA Ligase buffer"(10 % w/v) and "T4 DNA Ligase"(5 % w/v) (New England Biolabs GmbH (Frankfurt am Main, Germany). The ligation mix was incubated for 1 h at room temperature (RT).

Colonies that were grown on the transformation plates were verified with a colony PCR. 20 µl of the PCR reaction mix was used for each reaction tube. Usually 12 to 24 colonies were picked with a sterile pipette tip, transferred first to a well labelled master plate and afterwards to the reaction tube.

PCR was performed as described but with 10 min in the first 98° C step (to lyse the cells). 5 µl of PCR product then was loaded on an agarose gel and verified by gel electrophoresis.

**Table S1: Primers**

| List of primers that were designed and/or used in this work. Restriction sites are shown in red, linkers are shown in blue, start and stop sequences are shown in purple. | |
|---|---|
| Primer | Sequence from 5' to 3' |
| **AD321_SctQ_1fwd** | GACTGGGCCCCTTACCTGAATTGGGGGCTA |
| **AD324_SctQ_2rev** | GACTTCTAGAAGAGAATGGAGCCCCTAGTAAG |
| **AD339_pBAD_seq_fwd** | ATGCCATAGCATTTTTATCC |
| **AD340_pBAD_seq_rev** | GCGTTCTGATTTAATCTGTATCAGG |
| **AD341_pKNG_seq_fwd** | TATTAATTGATCTGCATCAACTTAACG |
| **AD342_pKNG_seq_rev** | GACTATACTAGTATACTCCGTCTACTGTACG |
| **AD638_ext_TatA_TMH_f** | GGTCTCCCATGGGTGGTATCAGTATTTGGCAGTTATT GATTATTGCCGTCATCGTTGTACTGCTTGTCCTATTTG GCACCAAAAAGCTCGGCTCCGACTACAAGGACGACG ATGATAAGGGTGGAGCAGGT |
| **AD642_MAG_r** | GACTGAATTC*TTA*CTCAGTCTCGCACTGAAACC |
| **AD698_Zdk1-EGFP_1fw** | GACTGGATCCTTGACTGAATGGTGGATAACAAATTCAA TAAAGAAAAGA |
| **AD699_Zdk1-EGFP_1rv** | ACCACCAGAGCCGCCCGACCCACCAGAACCACCTTTT GGGGCCT |
| **AD700_Zdk1-EGFP_2fw** | GGTGGGTCGGGCGGCTCTGGTGGTGGTGCTGGCGT GAGCAAG |
| **AD701_Zdk1-EGFP_2rv** | GATCGTCGAC*TTA*CTTGTACAGCTCGTCCATGC |
| **AD702_nMagHigh1-EGFP_fw** | GACTGATATCTGACTGAATGGGACACACTCTTTACGC CC |
| **AD703_nMagHigh1-EGFP_rev** | GCTAGGATCC*CTA*GTACAGCTCGTCCATTCCGA |
| **AD704_mCh-LOV2_int_fw** | GACGATGATAAGGGTGGAGCAGGTGTGAGCAAGGGC GAGGAG |
| **AD705_mCh-LOV2_rev** | GACTGAATTCGCAAGCTT*TTA*AAGTTCTTTTG |
| **AD706_pMAGF2-mCh_int_1fw** | GACGATGATAAGGGTGGAGCAGGTGTGAGCAAGGGC GAGGAG |
| **AD707_pMAGF2-mCh_1rv** | TCCACCTGCTCCACCACCAGCGCCCTTGTACAG |
| **AD708_pMAGF2-mCh_2fw** | GGCGCTGGTGGTGGAGCAGGTGGACACACTCTTTAC GCCCCTGG |
| **AD717_pACYC184_fw** | CAGGCACCGTGTATGAAATC |
| **AD718_pACYC184_rev** | GAGCCCGATCTTCCCCATC |
| **AD719_pACYC184_rev _SalI** | GTCCTCGCCGAAAATGACC |
| **AD720_pFL103_seq** | CTTACGGAAAGCTGACCCTG |
| **AD721_mCh_fwd** | GGTGGGTCGGGCGGCTCTGGTGGTGTGAGCAAGGG |
| | CGAGGAG |
| **AD722_mCh_rev** | GATCGTCGAC*TTA*CTTGTACAGCTCGTCCATGC |
| **AD723_nMagHigh1_rev2** | ACCACCAGAGCCGCCCGACCCACCTTCGGTTTCGCA CTGGAAT |
| **AD724_mCh_rev_Eagl** | GATCCGGCCG*TTA*CTTGTACAGCTCGTCCATGC |
| **AD732_ILID_mCh_2fw** | GGCGCTGGTGGTGGAGCAGGTGGAGGATCCGGGGA GTTTCTGG |
| **AD733_ILID_mCh_2rev** | GACTGAATTC*TCA*GCTAATTAAGCTTTTAAAAGT |
| **AD734_ILID_fw** | GACGATGATAAGGGTGGAGCAGGTGGATCCGGGGAG TTTCTGG |
| **AD735_SspB_Nano_fw** | GACTGGATCCTTGACTGAATGAGCTCCCCGAAACGCC C |
| **AD736_SspB_Nano_rev** | ACCACCAGAGCCGCCCGACCCACCACCAATATTCAGC TCGTCAT |
| **AD737_pACYC184_rev_ Eagl** | CCGGAAGCGAGAAGAATCATA |
| **AD759_Zdk1_premut_fwd** | GACTAGATCTGGCGCAGGTGTGGATAACAAATTCAAT AAAGAAAAGA |
| **AD762_SspB_premut_fwd** | GACTAGATCTGGCGCAGGTAGCTCCCCGAAACGCCC TAA |
| **AD768_mCh_premut_rev_EcoRI** | GACTGAATTCACCTGCGCCCTTGTACAGCTCGTCCAT GC |
| **AD769_SspB_premut_rev_EcoRI** | GACTGAATTCACCTGCGCCACCAATATTCAGCTCGTC ATAGA |
| **AD903_MA_LOV2_pACYC184_rev** | GACTGTCGACGCAAGCTT*TTA*AAGTTCTTTTG |
| **AD904_MA_iLID_pACYC184_rev** | GACTGTCGAC*TCA*GCTAATTAAGCTTTTAAAAGT |
| **AD921_MA_pACYC_fw_new** | GACTAGATCTTTGACTGAATGGGTGGTATCAGTATTTG GC |

### Transformation of Escherichia coli and Yersinia enterocolitica

Transformation of *E. coli* was either performed with *Top10* (strain for plasmid propagation) or with *Sm10 λpir*⁺ (strain that contains *pir* gene for pKNG101 propagation - pKNG101 can only replicate if π is provided in trans (as in the *E. coli Sm10λpir*⁺ strain) or if it integrates into the host chromosome (or pYVplasmid in *Yersinia*) (Kaniga *et al,* 1991) - used for 2-Step homologous recombination). For transformation of chemical competent *E. coli* (were made competent with TSS buffer (tryptone (1 % w/v), yeast extract (0.5 % w/v), NaCl (1 % w/v), PEG 3350 (10 % w/v), DMSO (5 % w/v), MgCl₂ (50 mM), pH = 6.5 - protocol adapted from (Chung & Miller, 1993)), 15 µl of ligation mix was added to the defrosted *E. coli* cells and incubated on ice for at least 30 min. The cells were then heat shocked for 1 min at 42° C water bath, incubated for 1 min on ice and were resuspended in 800 µl LB and incubated for 1 h at 37° C shaker (800 rpm). After incubation, the cells were spun down for 2 min and 8.000 rcf and resuspended in 50 µl remaining supernatant- the rest was discarded. 20 µl were plated on LB-plates with corresponding antibiotics and incubated at 37° C o/n.

Transformation of *Y. enterocolitica* was performed with *dHOPEMTasd.* For transformation of electro competent *Y. enterocolitica,* 1-2 µl of miniprep plasmid DNA was added to the defrosted *Y. enterocolitica* cells and incubated on ice for at least 15 min. The cells were then transferred into pre-cooled electroporation cuvettes and electroporated with a micropulser and the setting Ec2 (2.5 kV). Directly afterwards, cells were resuspended in 800 µl BHI + DAP (60 µg/ml) and transferred into new tubes. After incubating for 2 h at 28° C shaker (700 rpm) the cells were spun down for 2 min and 8.000 rcf and resuspended in 50 µl of remaining supernatant - the rest was discarded. 50 µl were plated on BHI + NAL + DAP + corresponding antibiotics and incubated at 28° C for 2-3 days.

### Strain construction by allelic exchange

For allelilc exchange by two-step homologous recombination, an o/n culture (2.5 ml of media + corresponding ingredients) of the acceptor strain (*Yersinia*) and the mutator strain (*E.coli* - *SM10λpir*⁺) were grown. 1 ml of o/n culture was spun down for 2 min at 10.000 rcf, the pellet was resuspended in 1 ml LB + DAP and spun down again. The pellet then was resuspended in 100 µl LB + DAP and 20 µl of the acceptor strain and the mutator strain were mixed in a sterile Eppendorf tube. 20 µl of the mix was spotted on a LB + DAP plate and incubated at 28° C for 4 h. After incubation, the grown spot was scratched and resuspended in 1 ml LB + DAP. 20 µl of the resuspended bacteria were plated on a LB + DAP + Nal + Sm (Sm selects for the first recombination step - integration of the mutator plasmid "PKNG101+Mutation" with a Sm-resistance into the pYV plasmid of *Yersinia* (Kaniga *et al*, 1991)). Then they were incubated for 2-3 days at 28° C. From single grown colonies, 6-8 were inoculated in 2.5 ml of BHI + Nal + DAP + Sm and cultivated o/n at 28° C on a shaker. 1.5 µl of the o/n culture were transferred into fresh tubes containing 2.5 ml of BHI + Nal + DAP and cultures were grown for at least 8 h at 28° C on a shaker (media is without Sm to initiate the second recombination step - the removal of the mutator plasmid (Kaniga *et al,* 1991)). After 8 h of incubation, 1.5 µl of culture were transferred into new tubes containing fresh 2.5 ml BHI+ Nal + DAP and incubated o/n at 28° C on a shaker. 20 µl of a 1:10 dilution of the o/n culture were plated on BHI + Nal + DAP + sucrose (8 % w/v) (sucrose selects for the absence of the mutator plasmid (Kaniga *et al*, 1991)) and incubated o/n at 28° C. The next day, a colony PCR was performed on single colonies to check for site directed mutagenesis.

### Analysis of protein expression and secretion activity

After induction of T3SS (4.3) 2 ml of bacteria culture was spun down for 10 min at 4° C and 12.000 rcf while measuring the OD₆₀₀ of the cell cultures to use for later calculations. For visualization of secreted proteins, 1.8 ml of the supernatant was mixed with 200 µl TCA (TCA is used for protein precipitation (Link & LaBaer, 2011)). After centrifugation for 15 min at 4° C and 20.000 rcf, the protein pellet was washed twice with 900 µl ice-cold acetone and spin down for 5 min at 4° C and max. speed inbetween and then could be used for further analysis. If the expressed T3SS proteins wanted to be quantified, the total cell pellet without supernatant was used for further analysis. For normalization of cell density, the pellet then was resuspended in calculated amount of 1x sample buffer (SDS (2 % w/v), Tris (0.1 M), glycerol (10 % w/v), DTT (0.05 M, pH = 6.8).

After heating the sample for 5 min at 99° C, 15 µl were loaded on a SDS-gel and run for 45-90 min at 130 V and 40 mA. The SDS-gel was then stained with staining solution for an optional time length (depends on how strong the colorizing effect should be) or used for western blot.

The SDS-gel was blotted on a nitrocellulose membrane using a Blot Transfer-system with the settings: 1.3 A, 25 V, 7 min. After blotting, the membrane was put in 15 ml milk solution (5 % w/v nonfat dried milk powder (PanReac AppliChem ITW Reagents (Darmstadt, Germany)) in 1x PBS (NaCl (137 mM), KCI (2.7 mM), Na₂HPO₄ (10 mM), KH₂PO₄ (2 mM), pH = 7.4)) and incubated o/n at 4° C on a shaker. The blot was washed once with 1x PBS and then incubated with the first antibody (diluted in milk solution (5 % w/v)) for 1 h at RT on a shaker.

Then the blot was washed 1x with 1x PBS, 1x with 1x PBS-T (1x PBS + Tween 20 (0.2 % w/v)), 1x with 1x PBS (washing steps always were performed for 1 min). After washing, the blot was incubated with the second antibody (diluted in milk solution (5 % w/v)) for 1 h at RT on a shaker. Then the blot was washed again 1x with 1x PBS, 4x with 1x PBS-T, 1x with 1x PBS. After removing the 1x PBS buffer, 800 µl of detection reagent ("Luminata™ Forte Western HRP Substrate" - MERCK (Darmstadt, Germany)) was added to the blot and spreaded evenly with a Drigalski spatula. Pictures of the blot were taken with a Luminescent Image Analyzer.

### Cell fixation

Cell fixation was performed after optogenetic cell cultivation. "Blue light" samples were incubated and handled under blue light, "dark" samples were incubated in the dark and handled under red light to avoid activation of the optogenetic system. 300 µl of bacterial culture were transferred in a tube containing 100 µl PFA (16 % w/v PFA in 1x PBS) and were incubated for 10-15 min. Cells were spun down for 4 min at 2.400 rcf and the pellet was washed afterwards 1x with Glycine (2 % w/v in 1x PBS) and 1x with 1x PBS. After fixation, cells could be stored at 4° for several days and used for fluorescence microscopy.

### Chemicals and online tools

Chemicals that were used for buffers or cultivation media were purchased from CARL ROTH GmbH & CO KG (Karlsruhe, Germany), SIGMA-ALDRICH (Steinheim, Germany), VWR Chemicals (Darmstadt, Germany) and Becton, Dickinson and Company (New Jersey, USA). All buffers, dNTP's, restriction enzymes and polymerases were purchased from THERMO FISHER SCIENTIFIC (Schwerte, Germany), CARL ROTH GmbH & CO KG (Karlsruhe, Germany) and New England Biolabs GmbH (Frankfurt am Main, Germany). For gel electrophoresis a "Quick-Load® Purple 2-Log DNA Ladder" (New England Biolabs GmbH) was used. For SDS-PAGE "Mini-PROTEAN® Precast Gels" (Life Science Research - BIO-RAD (California, USA)) and a "BlueClassic Prestained Protein Marker®" (Jena Bioscience (Jena, Germany)) were used. For staining of a SDS-Gel, an "Instant Blue staining solution" (Expedeon Inc. (San Diego, USA)) was used. For PCR purification and gel extraction a "NucleoSpin® Gel and PCR Clean-up" kit and for plasmid purification of *E. coli* a "NucleoSpin® Plasmid" kit (MACHEREY-NAGEL (Düren, Germany)) was used. For Western blot method a "Trans-Blot Turbo® Nitrocellulose- or PVDF-transfer pack" and a Trans-Blot Turbo® Transfer System (Life Science Research - BIO-RAD (California, USA)) were used. Pictures of the blot were taken on a "Luminescent Image Analyzer Las-4000 (Fujifilm (Minato, J) with the corresponding software "ImageReader LAS-4000". Antibodies were purchased from THERMO FISHER SCIENTIFIC (Schwerte, Germany). Measurements of DNA-concentration or optical density (OD₆₀₀) of cell cultures were performed on a "DS-11 + Spectrophotometer" (DeNovix Inc. (Wilmington, USA)). Electroporation of *Yersinia* cells were performed with a "MicroPulser™ Electroporator" and "Gene *E. coli* Pulser Cuvettes" 0.2 cm (Life Science Research - BIO-RAD (California, USA)). During fluorescence microscopy the images were taken on a Deltavision Spectris Optical Sectioning Microscope (Applied Precision, Issaquah, WA, USA), equipped with a UPlanSApo x 100/1.40 oil objective (Olympus, Tokyo, Japan) and x 1.6 auxiliary magnification, using an Evolve EMCCD Camera (Photometrics, Tucson, AZ, USA) at a gain level 50. Microscopy pictures were analyzed and processed with ImageJ-Fiji (Schindelin *et al,* 2012). All primers and sequencing were placed in order at EUROFINS GENOMICS (Ebersberg, Germany). Gene sequences were bioinformatically analyzed and designed with SerialCloner 2.6.1 (Serial Basics) and the online tools listed in Table S2. Primers that were designed and used during this work are listed in Table S1.

**Table S2: Online Tools**

| List of online tools that were used for sequence analysis and primer design. | |
|---|---|
| Name | URL |
| **Expasy Translate tool** | https://web.expasy.org/translate/ |
| **WebCutter** | http://rna.lundberg.gu.se/cutter2/ |
| **Primer 3** | http://primer3.ut.ee/ |
| **Nucleic Acid Sequence Massager** | http://www.attotron.com/cybertory/analysis/seqMassager. htm |
| **NEB cloner** | https://nebcloner.neb.com/#!/ |

### REFERENCES

Bai F, Li Z, Umezawa A, Terada N & Jin S (2018) Bacterial type III secretion system as a protein delivery tool for a broad range of biomedical applications. Biotechnol. Adv. 36: 482-493
Biemans-Oldehinkel E, Sal-Man N, Deng W, Foster LJ & Finlay BB (2011) Quantitative proteomic analysis reveals formation of an EscL-EscQ-EscN type III complex in enteropathogenic Escherichia coli. J. Bacteriol. 193: 5514-9
Blanco-Toribio A, Muyldermans S, Frankel G & Fernández LÁ (2010) Direct Injection of Functional Single-Domain Antibodies from E. coli into Human Cells. PLoS One 5: e15227
Cornelis GR (2002) The Yersinia Ysc-Yop 'type III' weaponry. Nat. Rev. Mol. Cell Biol. 3: 742-52
Cornelis GR (2006) The type III secretion injectisome. Nat. Rev. Microbiol. 4: 811-825 Deisseroth K (2011) Optogenetics. Nat. Methods 8: 26-29
Deng W, Marshall NC, Rowland JL, McCoy JM, Worrall LJ, Santos AS, Strynadka NCJ & Finlay BB (2017) Assembly, structure, function and regulation of type III secretion systems. Nat. Rev. Microbiol.
Diepold A, Amstutz M, Abel S, Sorg I, Jenal U & Cornelis GR (2010) Deciphering the assembly of the Yersinia type III secretion injectisome. EMBO J. 29: 1928-1940 Diepold A & Armitage JP (2015) Type III secretion systems: the bacterial flagellum and the injectisome. Philos. Trans. R. Soc. B Biol. Sci. 370: 20150020
Diepold A, Kudryashev M, Delalez NJ, Berry RM & Armitage JP (2015) Composition, Formation, and Regulation of the Cytosolic C-ring, a Dynamic Component of the Type III Secretion Injectisome. PLOS Biol. 13: e1002039
Diepold A, Sezgin E, Huseyin M, Mortimer T, Eggeling C & Armitage JP (2017) A dynamic and adaptive network of cytosolic interactions governs protein export by the T3SS injectisome. Nat. Commun. 8: 15940
Diepold A & Wagner S (2014) Assembly of the bacterial type III secretion machinery. FEMS Microbiol. Rev. 38: 802-22
Diepold A, Wiesand U & Cornelis GR (2011) The assembly of the export apparatus (YscR,S,T,U,V) of the Yersinia type III secretion apparatus occurs independently of other structural components and involves the formation of an YscV oligomer. Mol. Microbiol. 82: 502-14
Enninga J, Mounier J, Sansonetti P, Tran Van Nhieu G & Nhieu GT Van (2005) Secretion of type III effectors into host cells in real time. Nat. Methods 2: 959-65 Felgner S, Pawar V, Kocijancic D, Erhardt M & Weiss S (2017) Tumour-targeting bacteria-based cancer therapies for increased specificity and improved outcome. Microb. Biotechnol. 10: 1074-1078
Gambetta, G. A. and Lagarias, J. C. (2001) 'Genetic engineering of phytochrome biosynthesis in bacteria.', Proceedings of the National Academy of Sciences of the United States of America. National Academy of Sciences, 98(19), pp. 10566-71. doi: 10.1073/pnas. 191375198.
Gauthier A & Finlay BB (2003) Translocated intimin receptor and its chaperone interact with ATPase of the type III secretion apparatus of enteropathogenic Escherichia coli. J. Bacteriol. 185: 6747-55
Guntas G, Hallett RA, Zimmerman SP, Williams T, Yumerefendi H, Bear JE & Kuhlman B (2015) Engineering an improved light-induced dimer (iLID) for controlling the localization and activity of signaling proteins. Proc. Natl. Acad. Sci. 112: 112-117
Hu B, Lara-Tejero M, Kong Q, Galán JE & Liu J (2017) In Situ Molecular Architecture of the Salmonella Type III Secretion Machine. Cell 168: 1065-1074.e10
Hueck CJ (1998) Type III protein secretion systems in bacterial pathogens of animals and plants. Microbiol. Mol. Biol. Rev. 62: 379-433
Ittig SJ, Schmutz C, Kasper CA, Amstutz M, Schmidt A, Sauteur L, Vigano MA, Low SH, Affolter M, Cornelis GR, Nigg EA & Arrieumerlou C (2015) A bacterial type III secretion-based protein delivery tool for broad applications in cell biology. J. Cell Biol. 211: 913-31 Jacobi CA, Roggenkamp A, Rakin A, Zumbihl R, Leitritz L & Heesemann J (1998) In vitro and in vivo expression studies of yopE from Yersinia enterocolitica using the gfp reporter gene. Mol. Microbiol. 30: 865-882
Jayaraman P, Devarajan K, Chua TK, Zhang H, Gunawan E & Poh CL (2016) Blue light-mediated transcriptional activation and repression of gene expression in bacteria. Nucleic Acids Res. 44: 6994-7005
Johnson S & Blocker AJ (2008) Characterization of soluble complexes of the Shigella flexneri type III secretion system ATPase. FEMS Microbiol. Lett. 286: 274-8 Kaberniuk AA, Shemetov AA & Verkhusha V V (2016) A bacterial phytochrome-based optogenetic system controllable with near-infrared light. Nat. Methods 13: 591-7 Kaniga K, Delor I & Cornelis GR (1991) A wide-host-range suicide vector for improving reverse genetics in gram-negative bacteria: inactivation of the blaA gene of Yersinia enterocolitica. Gene 109: 137-41
Kawano F, Aono Y, Suzuki H & Sato M (2013) Fluorescence Imaging-Based High-Throughput Screening of Fast- and Slow-Cycling LOV Proteins. PLoS One 8: e82693 Kawano F, Suzuki H, Furuya A & Sato M (2015) Engineered pairs of distinct photoswitches for optogenetic control of cellular proteins. Nat. Commun. 6: 6256 Kudryashev M, Stenta M, Schmelz S, Amstutz M, Wiesand U, Castaño-Díez D, Degiacomi MT, Münnich S, Bleck CK, Kowal J, Diepold A, Heinz DW, Dal Peraro M, Cornelis GR & Stahlberg H (2013) In situ structural analysis of the Yersinia enterocolitica injectisome. Elife 2: e00792
Lambert de Rouvroit C, Sluiters C & Cornelis GR (1992) Role of the transcriptional activator, VirF, and temperature in the expression of the pYV plasmid genes of Yersinia enterocolitica. Mol. Microbiol. 6: 395-409
Lara-Tejero M, Kato J, Wagner S, Liu X & Galán JE (2011) A Sorting Platform Determines the Order of Protein Secretion in Bacterial Type III Systems. Science (80-.). 331: 1188-91
Lara-Tejero M, Qin Z, Hu B, Butan C, Liu J & Galán JE (2019) Role of SpaO in the assembly of the sorting platform of a Salmonella type III secretion system. PLOS Pathog. 15: e1007565
Michiels T, Wattiau P, Brasseur R, Ruysschaert JM & Cornelis GR (1990) Secretion of Yop proteins by Yersiniae. Infect. Immun. 58: 2840-9
Mills E, Baruch K, Charpentier X, Kobi S & Rosenshine I (2008) Real-time analysis of effector translocation by the type III secretion system of enteropathogenic Escherichia coli. Cell Host Microbe 3: 104-13
Morita-ishihara T, Ogawa M, Sagara H, Yoshida M, Katayama E & Sasakawa C (2005) Shigella Spa33 is an essential C-ring component of type III secretion machinery. J. Biol. Chem. 281: 599-607
Mukherjee A, Repina NA, Schaffer D V & Kane RS (2017) Optogenetic tools for cell biological applications. J. Thorac. Dis. 9: 4867-4870
Palmer T & Berks BC (2012) The twin-arginine translocation (Tat) protein export pathway. Nat. Rev. Microbiol. 10: 483-496
Pathak, G. P., Strickland, D., Vrana, J. D. and Tucker, C. L. (2014) 'Benchmarking of optical dimerizer systems.', ACS synthetic biology. American Chemical Society, 3(11), pp. 832-8. doi: 10.1021/sb500291r.
Pettersson J, Nordfelth R, Dubinina E, Bergman T, Gustafsson M, Magnusson KE & Wolf-Watz H (1996) Modulation of virulence factor expression by pathogen target cell contact. Science (80-.). 273: 1231-3
Reichhart E, Ingles-Prieto A, Tichy A-M, McKenzie C & Janovjak H (2016) A Phytochrome Sensory Domain Permits Receptor Activation by Red Light. Angew. Chemie Int. Ed. 55: 6339-6342
Schlumberger MC, Müller A, Ehrbar K, Winnen B, Duss I, Stecher B, Hardt W-D & Mu AJ (2005) Real-time imaging of type III secretion: Salmonella SipA injection into host cells. Proc. Natl. Acad. Sci. U. S. A. 102: 12548-12553
Schraidt O & Marlovits TC (2011) Three-dimensional model of Salmonella's needle complex at subnanometer resolution. Science (80-.). 331: 1192-5
Schulte M, Sterzenbach T, Miskiewicz K, Elpers L, Hensel M & Hansmeier N (2018) A versatile remote control system for functional expression of bacterial virulence genes based on the tetA promoter. Int. J. Med. Microbiol.
Shi L, Yu B, Cai C-H & Huang J-D (2016) Angiogenic inhibitors delivered by the type III secretion system of tumor-targeting Salmonella typhimurium safely shrink tumors in mice. AMB Express 6: 56
Shimizu-Sato S, Huq E, Tepperman JM & Quail PH (2002) A light-switchable gene promoter system. Nat. Biotechnol. 20: 1041-1044
Song M, Sukovich DJ, Ciccarelli L, Mayr J, Fernandez-Rodriguez J, Mirsky EA, Tucker AC, Gordon DB, Marlovits TC & Voigt CA (2017) Control of type III protein secretion using a minimal genetic system. Nat. Commun. 8: 14737
Sory M-PP, Boland A, Lambermont I & Cornelis GR (1995) Identification of the YopE and YopH domains required for secretion and internalization into the cytosol of macrophages, using the cyaA gene fusion approach. Proc. Natl. Acad. Sci. U. S. A. 92: 11998-12002
Toettcher JE, Voigt CA, Weiner OD & Lim WA (2011) The promise of optogenetics in cell biology: interrogating molecular circuits in space and time. Nat. Methods 8: 35-8 [*Toettcher et al, 2011a*]*.*
Toettcher, J. E., Gong, D., Lim, W. A. and Weiner, O. D. (2011) 'Light control of plasma membrane recruitment using the Phy-PIF system.', Methods in enzymology. NIH Public Access, 497, pp. 409-23. doi: 10.1016/B978-0-12-385075-1.00017-2 [*Toettcher et al, 2011b*]*.*
Wagner S, Grin I, Malmsheimer S, Singh N, Torres-Vargas CE & Westerhausen S (2018) Bacterial type III secretion systems: A complex device for delivery of bacterial effector proteins into eukaryotic host cells. FEMS Microbiol. Lett.
Walker BJ, Stan G-B V. & Polizzi KM (2017) Intracellular delivery of biologic therapeutics by bacterial secretion systems. Expert Rev. Mol. Med. 19: e6
Wang H, Vilela M, Winkler A, Tarnawski M, Schlichting I, Yumerefendi H, Kuhlman B, Liu R, Danuser G & Hahn KM (2016) LOVTRAP: an optogenetic system for photoinduced protein dissociation. Nat. Methods 13: 755-8
Wattiau P & Cornelis GR (1993) SycE, a chaperone-like protein of Yersinia enterocolitica involved in Ohe secretion of YopE. Mol. Microbiol. 8: 123-31
Zhang Y, Lara-Tejero M, Bewersdorf J & Galán JE (2017) Visualization and characterization of individual type III protein secretion machines in live bacteria. Proc. Natl. Acad. Sci. U. S. A. 114: 6098-6103
Zimmerman SP, Hallett RA, Bourke AM, Bear JE, Kennedy MJ & Kuhlman B (2016) Tuning the Binding Affinities and Reversion Kinetics of a Light Inducible Dimer Allows Control of Transmembrane Protein Localization. Biochemistry 55: 5264-71

## Claims

1. A recombinant gram-negative bacterium comprising a type III secretion system, wherein said type III secretion system is light-dependent, wherein said recombinant gram-negative bacterium comprises an optogenetic interaction switch.

2. The recombinant gram-negative bacterium of claim 1, which expresses at least one recombinant protein comprising (i) a cargo protein to be secreted by said type III secretion system and (ii) a secretion signal of said type III secretion system.

3. The recombinant gram-negative bacterium of claim 1 or 2, wherein said optogenetic interaction switch comprises a first and a second fusion protein, which specifically bind to each other in a light-dependent way.

4. The recombinant gram-negative bacterium of any one of claims 1 to 3, wherein said recombinant gram-negative bacterium expresses (a) a first fusion protein comprising (aa) a cytosolic component of said type III secretion system, and (ab) a first component of said optogenetic interaction switch, and (b) a second fusion protein comprising (ba) an inner membrane anchor protein and (bb) a second component of said optogenetic interaction switch, wherein said first component of said optogenetic interaction switch and said second component of said optogenetic interaction switch specifically bind to each other in a light-dependent way.

5. The recombinant gram-negative bacterium of claim 4, wherein said first fusion protein is expressed from a first nucleic acid sequence operably linked to first expression control sequences, and said second fusion protein is expressed from a second nucleic acid sequence operably linked to second expression control sequences, wherein expression of said first fusion protein is lower than expression of said second fusion protein, particularly lower by a factor of at least two, more particularly lower by a factor of at least five,
particularly where said cytosolic component is a component of said type III secretion system with native low expression and/or low stoichiometry, and/or wherein said first nucleic acid sequence is either expressed from an inducible promoter or replaces the native nucleic acid sequence encoding said cytosolic component on the virulence plasmid or in the virulence region on the bacterial genome.

6. The recombinant gram-negative bacterium of any one of claims 1 to 5, wherein said recombinant gram-negative bacterium is selected from *Yersinia enterocolitica and Pseudomonas aeruginosa.*

7. The recombinant gram-negative bacterium of claim 6, wherein said recombinant gram-negative bacterium is selected from *Yersinia enterocolitica,* particularly wherein the six main virulence effectors of *Yersinia enterocolitica* have been deleted, more particularly wherein said recombinant gram-negative bacterium is from strain IML421asd.

8. The recombinant gram-negative bacterium of any one of claims 1 to 7, wherein the type III secretion system is functionally inactive in the absence of light of a particular wavelength, and can be functionally activated by illumination with light of said wavelength, particularly wherein said optogenetic interaction switch is the LOV switch, or an optogenetic interaction switch derived therefrom, more particularly wherein said first component of said optogenetic interaction switch is Zdk1, particularly Zdk1 according to Addgene No. 81010, and said second component of said optogenetic interaction switch is LOV2 particularly LOV2 according to Addgene No. 81041, or the V416L point mutation thereof.

9. The recombinant gram-negative bacterium of any one of claims 1 to 7, wherein the type III secretion system is functionally inactive in the presence of light of a particular wavelength, and can be functionally activated by removing illumination with light of said wavelength, particularly wherein said optogenetic interaction switch is the Magnet switch, or an optogenetic interaction switch derived therefrom, more particularly wherein said first component of said optogenetic interaction switch is nMAGHigh1, particularly nMAGHigh1 according to Addgene No. 67300, and said second component of said optogenetic interaction switch is pMAGFast2(3x), particularly pMAGFast2(3x)* according to Addgene No. 67297, or a variant of pMAGFast2(3x)* with two instead of three repeats of the domain.

10. The recombinant gram-negative bacterium of any one of claims 1 to 7, wherein the type III secretion system is functionally inactive in the presence of light of a particular wavelength, and can be functionally activated by removing illumination with light of said wavelength, particularly wherein said optogenetic interaction switch is the iLID switch, or an optogenetic interaction switch derived therefrom, more particularly wherein said first component of said optogenetic interaction switch is SspB, particularly SspB_Nano according to Addgene No. 60409, and said second component of said optogenetic interaction switch is iLID particularly iLID according to Addgene No. 60408, or the C530M point mutation thereof.

11. The recombinant gram-negative bacterium of any one of claims 1 to 7, wherein the type III secretion system is functionally inactive in the presence of light of a particular first wavelength, and is functionally active in the presence of light of a particular second wavelength, particularly wherein said optogenetic interaction switch is the Phy-PIF switch, more particularly wherein said first component of said optogenetic interaction switch is a fragment of a phytochrome interaction factor protein (PIF), particularly a PIF fragment consisting of residues 1-100 of *A*. *thaliana* PIF6 protein, and said second component of said optogenetic interaction switch is Phy, particularly a Phy variant consisting of residues 1-908 of the *A*. *thaliana* PhyB protein.

12. A method for modifying the translocation of one or more cargo proteins from a recombinant gram-negative bacterium, comprising the steps of (i) culturing a recombinant gram-negative bacterium comprising a light-dependent type III secretion system of any one of claims 1 to 11 under a first light condition, and (ii) culturing said recombinant gram-negative bacterium under a second light condition, wherein the change from said first light condition to said second light condition modifies the translocation activity of said light-dependent type III secretion system.

13. The method of claim 12, wherein said translocation activity is secretion of said one or more cargo proteins into the culture medium.

14. The method of claim 12, wherein said translocation activity is transfer of said one or more cargo proteins into a eukaryotic host cell.

15. The recombinant gram-negative bacterium of any one of claims 1 to 3, wherein the bacterium expresses (a) a first fusion protein comprising (aa) a secretion signal, and (ab) a first component of said optogenetic interaction switch, and (ac) a cargo protein to be translocated by the type III secretion system, and (b) a second fusion protein comprising (ba) an inner membrane anchor protein and (bb) a second component of said optogenetic interaction switch, wherein said first component of said optogenetic interaction switch and said second component of said optogenetic interaction switch specifically bind to each other in a light-dependent way.
